# EUROPÄISCHE PATENTSCHRIFT

(11) **EP 1 385 975 B1**
(45) Veröffentlichungstag und Bekanntmachung des Hinweises auf die Patenterteilung: **03.01.2007**
(21) Anmeldenummer: 02742430.8
(22) Anmeldetag: 20.02.2002
(51) Int. Cl.: C12P 13/02, A23K 1/00

(54) **VERFAHREN ZUR HERSTELLUNG VON D-PANTOTHENSÄURE UND/ODER SALZE ALS ZUSATZ ZU TIERFUTTERMITTELN**
METHOD FOR THE PRODUCTION OF D-PANTOTHENIC ACID AND/OR SALTS THEREOF AS ADJUNCT FOR ANIMAL FEEDSTUFFS
PROCEDE DE PRODUCTION D'ACIDE D-PANTOTHENIQUE ET/OU DE SELS DE CET ACIDE COMME COMPLEMENTS D'ALIMENTS POUR ANIMAUX

(30) Priorität: 21.02.2001 DE 10108226
(43) Veröffentlichungstag der Anmeldung: 04.02.2004
(73) Patentinhaber: BASF Aktiengesellschaft, 67056 Ludwigshafen (DE)
(72) Erfinder: BECK, Christine, 68167 Mannheim (DE); HARZ, Hans-Peter, 67373 Dubenhofen (DE); KLEIN, Daniela, 68161 Mannheim (DE); LEEMANN, Martin, 64625 Bensheim (DE); LOHSCHEIDT, Markus, 68199 Mannheim (DE); BITTERLICH, Stefan, 67246 Dirmstein (DE); VOSS, Hartwig, 67227 Frankenthal (DE)
(74) Vertreter: Fitzner, Uwe
(86) Internationale Anmeldenummer: PCT/EP2002/001754
(87) Internationale Veröffentlichungsnummer: WO 2002/066664

(56) Entgegenhaltungen:
- EP-A- 0 590 857
- EP-A- 1 006 192
- EP-A- 1 050 219
- WO-A-01/21772
- WO-A-02/24001
- GB-A- 562 267
- BAIGORI M ET AL.: "Isolation and characterization of Bacillus subtilis mutants blocked in the synthesis of pantothenic acid" JOURNAL OF BACTERIOLOGY, Bd. 173, Nr. 13, Juli 1991 (1991-07), Seiten 4240-4242, XP001002216 ISSN: 0021-9193
- DATABASE EMBL [Online] EMBL; EMBL:BSYPIA, ID BSYPIA, AC L47709, 23. Januar 1996 (1996-01-23) HENNER D ET AL.: "Bacillus subtilis (clone YAC15-6B) ypiABF genes, qcrABC genes, ypjABCDEFGHI genes, birA gene, panBCD genes, dinG gene, ypmB gene, aspB gene, asnS gene, dnaD gene, nth gene and ypoC gene, complete cds" Database accession no. L47709 XP002171539
- BEGLEY T P ET AL.: "The biosynthesis of Coenzyme A in bacteria" VITAMINS AND HORMONES, ACADEMIC PRESS, NEW YORK, US, Bd. 61, Februar 2001 (2001-02), Seiten 157-171, XP008004305 ISSN: 0083-6729

## Beschreibung

Die vorliegende Erfindung betrifft ein verbessertes Verfahren zur Herstellung von D-Pantothensäure und/oder deren Salze und die Verwendung als Zusatz zu Tierfuttermitteln.

Als ein Ausgangsprodukt der Biosynthese von Coenzym A ist D-Pantothenat in der Pflanzen- und Tierwelt weit verbreitet. Im Gegensatz zum Menschen, der die Pantothensäure in ausreichenden Mengen über die Nahrung zu sich nimmt, sind jedoch sowohl für Pflanzen als auch für Tiere häufig Mangelerscheinungen für D-Pantothenat beschrieben. Die Verfügbarkeit von D-Pantothenat ist daher von großem wirtschaftlichen Interesse, insbesondere in der Tierfutterindustrie.

Herkömmlicher Weise erfolgt die Herstellung von D-Pantothenat durch chemische Synthese aus D-Pantolacton und Caicium-β-Alaninat (Ullmann's Encyclopedia of Industrial Chemistry, 6^{th} edition, 1999, electronic release, Kapitel "Vitamins"). Zur Bereitstellung von D-Pantolacton ist eine aufwendige, klassische Racematspaltung über diastereomere Salze erforderlich. Das aus der chemischen Synthese resultierende Verkaufsprodukt ist meist das Calciumsalz der D-Pantothensäure Calcium-D-Pantothenat.

Gegenüber der chemischen Synthese liegt der Vorteil biotechnologischer Herstellungsverfahren mit Mikroorganismen in der selektiven (enantiomerenreinen) Bereitstellung der für den höheren Organismus verwertbaren D-Form der Pantothensäure. Eine aufwendige Racematspaltung, wie sie bei der chemischen Synthese erforderlich ist, entfällt somit.

Fermentative Verfahren zur Herstellung von D-Pantothensäure mit Mikroorganismen sind zahlreich bekannt, so u.a. aus EP 0 590 857, WO 96/33283, US 6,013,492, WO 97/10340, DE 198 46 499, EP 1 001 027, EP 1 006 189, EP 1 006 192 und EP 1 006 193.

So beschreiben EP 1 006 189 und EP 1 001 027 Verfahren zur Herstellung von Pantothenat bei dem in der Fermentationslösung ein Gehalt von höchstens 1 g/l D-Pantothensäure erreicht wird. Solche geringen Pantothensäure-Gehalte in der Fermentationslösung, also von weniger als 10 Gew.-% bezogen auf den Feststoffgehalt sind jedoch für eine wirtschaftliche Herstellung von D-Pantothensäure enthaltenden Tierfuttersupplementen ungeeignet. Ein weiterer Nachteil bei den bislang beschriebenen Verfahren ist, daß die Isolierung des Produkts aus dem Fermentationsmedium zahlreiche aufwendige Aufarbeitungsschritte erfordert. Ein wirtschaftliches Herstellungsverfahren für den großtechnischen Maßstab ist nicht offenbart.

In der Offenlegungsschrift DE 100 16 321 ist ein Fermentationsverfahren zur Herstellung eines D-Pantothensäure-haltigen Tierfuttersupplements beschrieben. Ein wesentlicher Nachteil diese Verfahrens ist jedoch, wie auch bei den oben angeführten fermentativen Verfahren zur D-Pantothensäure-Herstellung, daß dem Mikroorganismus über das Fermentationsmedium die Pantothensäure-Vorstufe β-Alanin zwingend zugeführt werden muß, um wirtschaftliche Ausbeuten des gewünschten Produktes zu erhalten.

Ferner beschreiben US 6,013,492 und WO 96/332839 die Aufarbeitung der D-Pantothensäure aus der Fermentationslösung durch Abfiltern unlöslicher Bestandteile (z. B. Zellmaterial) vom Kulturmedium, eine Adsorption des Filtrats an Aktivkohle, eine sich anschließende Elution der D-Pantothensäure mit einem organischen Lösungsmittel, bevorzugt Methanol, eine Neutralisation mit Calciumhydroxid und eine abschließende Kristallisation von Calcium-D-Pantothenat. Wesentliche Nachteile sind die bei der Kristallisation auftretenden Wertproduktverluste sowie die Verwendung eines organischen Lösungsmittels, das nur schwer aus dem Produkt zu entfernen ist und eine aufwendige Lösungsmittelrückgewinnung erforderlich macht.

Die EP 0 590 857 beschreibt ein Fermentationsverfahren zur Herstellung von D-Pantothensäure, bei dem die Kultivierung eines Mikroorganismus die Zufütterung von β-Alanin zwingend erfordert. Die Fermentationslösung wird zur Abtrennung der Biomasse filtriert, dann über einen Kationenaustauscher und anschließend über einen Anionenaustauscher geleitet, im Anschluß daran mit Calciumhydroxid neutralisiert, eingedampft, mit Aktivkohle versetzt, nochmals filtriert und unter Zusatz von Methanol und Calciumchlorid kristallisiert. Das resultierende Calciumpantothenat-haltige Produkt enthält neben D-Pantothensäure in Form des Caliumssalzes noch Calciumchlorid in einem Molverhältnis von 1:1. Zur Reduzierung des Calciumchloridgehaltes ist eine Elektrodialyse mit anschließender Sprühtrocknung erforderlich. Dieses Verfahren hat den Nachteil, wegen der Vielzahl aufwendiger Verfahrensschritte und der Verwendung organischer Lösungsmittel weder ökonomisch noch ökologisch zu sein.

Aufgabe der vorliegenden Erfindung ist die Bereitstellung eines D-Pantothensäure und/oder deren Salze enthaltendes Tierfuttersupplement sowie dessen Herstellung durch ein verbessertes Verfahren zur Herstellung von D-Pantothensäure und/oder deren Salzen, das die zuvor genannten Nachteile nicht aufweist. Hierbei ist aus wirtschaftlichen Gründen ein Verfahren wünschenswert, bei dem eine Zufütterung von β-Alanin drastisch reduziert oder überhaupt nicht erforderlich ist. Ferner ist die Herstellung von D-Pantothensäure in Form ihrer zweiwertigen Salze und hierbei vor allem der Erdalkali-Salze wünschenswert, da die zweiwertigen Salze weniger hygroskopische Eigenschaften aufweisen als einwertige Salze der Pantothensäure und für die weitere Verwendung, z. B. als Tierfuttersupplement, somit weniger stark zu Verklumpungen neigen.

Diese Aufgabe wird durch die vorliegende Erfindung in vorteilhafter Weise gelöst.

Gegenstand der vorliegenden Erfindung ist ein Verfahren zur Herstellung von D-Pantothensäure und/oder deren Salze, dadurch gekennzeichnet, daß
a) wenigstens ein D-Pantothensäure produzierender Mikroorganismus eingesetzt wird, dessen Pantothensäure-(pan)- und/oder Isoleucin/Valin-(ilv)-Biosynthese dereguliert ist und der wenigstens 2 g/l an Salzen der D-Pantothensäure durch Fermentation in einem Kulturmedium bildet, wobei dem Kulturmedium 0 - 20 g/l freies β-Alanin und/oder β-Alanin-Salz zugeführt wird,
b) dem gebildeten D-Pantothenat Salze enthaltend mehrwertige Kationen zugeführt werden, wobei mehrwertige Salze der D-Pantothensäure ausgebildet werden,
c) die Lösung enthaltend mehrwertige Salze der D-Pantothensäure durch Nanofiltration aufbereitet wird, wobei die mehrwertigen Salze der D-Pantothensäure angereichert werden und
d) das Retentat der Nanofiltration enthaltend mehrwertige Salze der D-Pantothensäure einer Trocknung und/oder Formulierung unterzogen wird.

In einer Variante des erfindungsgemäßen Verfahrens ist das Retentat aus Schritt c) eine Suspension enthaltend mehrwertige Salze der D-Pantothensäure.

Ferner kann die Fermentation nach an sich bekannten Vorgehensweisen im batch-, fed-batch- oder repeated-fed-batch-Betrieb oder bei kontinuierlicher Prozeßführung durchgeführt werden. Zur Neutralisation der entstehenden Pantothensäure werden hierbei übliche Puffersysteme, wie z. B. Phosphat-Puffer mit NaOH, KOH oder Ammoniak benutzt.

In weiteren Varianten des erfindungsgemäßen Verfahrens werden in Schritt a) wenigstens 10 g/l, bevorzugt wenigstens 20 g/l, besonders bevorzugt wenigstens 40 g/l, höchst besonders bevorzugt wenigstens 60 g/l und insbesondere wenigstens 70 g/l an Salzen der D-Pantothensäure durch Fermentation im Kulturmedium gebildet.

Erfindungsgemäß ist unter der Formulierung "produzieren" zu verstehen, daß der Mikroorganismus größere Mengen D-Pantothensäure und/oder deren Salze synthetisieren kann, als für den eigenen Stoffwechselbedarf erforderlich sind. In einer erfindungsgemäß vorteilhaften Variante liegt die synthetisierte Menge an D-Pantothensäure und/oder deren Salze nicht zellintern vor, sondern wird idealerweise vollständig aus dem Mikroorganismus in das Kulturmedium abgegeben. Dies Ausschleusung kann aktiv oder passiv nach an sich bekannten Mechanismen erfolgen.

Erfindungsgemäß werden als D-Pantothensäure produzierende Mikroorganismen Pilze, Hefen und/oder Bakterien eingesetzt. Erfindungsgemäß werden bevorzugt Pilze wie beispielsweise Mucor oder Hefen, wie z. B. Saccharomyces oder Debaromyces und hierbei bevorzugt Saccharaomyces cerevisiae eingesetzt. Vorteilhaft werden erfindungsgemäß coryneforme Bakterien oder Bacillaceae verwendet. Erfindungsgemäß umfaßt sind bevorzugt z. B. Bakterien der Gattungen Corynebacterium, Escherichia, Bacillus, Arthrobacter, Bevibacterium, Pseudomonas, Salmonella, Klebsiella, Proteus, Acinetobacter oder Rhizobium. Besonders bevorzugt sind hierbei beispielsweise Corynebacterium glutamicum, Brevibacterium breve oder Bacillus subtilis, B. licheniformis, B. amyloliquefaciens, B. cereus, B. lentimorbus, B. lentus, B. firmus, B.pantothenticus, B. circulans, B. coagulans, B. megaterium, B. pumilus, B. thuringiensis, B. brevis, B. stearothermophilus und andere Bacillusarten der Gruppe 1, die durch ihre 16sRNA charakerisiert sind oder Actinum mycetalis. Diese Aufzählung dient der Erläuterung und ist keinesfalls limitierend für die vorliegende Erfindung.

Darüber hinaus umfaßt die vorliegende Erfindung auch den Einsatz genetisch veränderter Mikroorganismen zur erfindungsgemäßen Herstellung eines Tierfuttersupplements enthaltend freie D-Pantothensäure und/oder deren Salze. Solche genetisch veränderten Mikroorganismen können beispielsweise durch chemische Mutagenese und anschließende Selektion durch eine geeignetes "Screeningverfahren" isoliert werden. Erfindungsgemäß sind auch sogenannte "Produktionsstämme" umfaßt, die zur Herstellung des Produktes im Sinne der vorliegenden Erfindung geeignet sind und genetische Veränderungen hinsichtlich des Stoffwechselflusses in Richtung D-Pantothensäure aufweisen, wobei auch Veränderungen hinsichtlich der Ausschleusung von D-Pantothensäure und/oder deren Salzen über die Zellmembran inbegriffen sind. Dies kann z. B. durch Veränderungen an Schlüsselpositionen in relevanten Stoffwechsel-Biosynthesewegen des eingesetzten Mikroorganismus erreicht werden.

Denkbar ist auch der Einsatz transgener Mikroorganismen, die aus der Übertragung homologer und/oder heterologer Nukleotidsequenzen resultieren, welche zur Synthese des gewünschten Produktes erforderlich sind oder förderlich sein können. Hierbei ist die Überexpression und/oder Deregulation ein oder mehrerer Gene einzeln und/oder in Kombination, lokalisiert im Genom und/oder auf einem Vektor, denkbar.
Derartige transgene Mikroorganismen können in vorteilhafter Weise zusätzliche Kopien und/oder genetisch veränderte Gene ausgewählt aus der Gruppe von panB, panC, panD, panE und/oder deren Kombinationen und/oder sogar Organisationseinheiten, wie das panBCD-Operon enthalten. Ferner können weitere Stoffwechselwege, wie z.B. der Isoleucin-Valin-Biosyntheseweg in den Mikroorganismen vorteilhaft manipuliert sein, wie beispielsweise in der EP 1 006 189, EP 1 006 192, EP 1 006 193 oder EP 1 001 027 beschrieben ist. Dadurch werden verzweigtkettige Vorläufersubstanzen der Pantothensäure-Biosynthese vermehrt zur Verfügung gestellt. Vorteilhaft werden gegebenenfalls die Gene für diesen Biosyntheseweg d.h. ilvB, ilvN, ilvC und/oder ilvD überexprimiert.
Darüber hinaus sind genetische Veränderungen der Aspartat-α-Decarboxylase (panD), z.B. durch Überexpression und/oder Deregulation, in dem eingesetzten D-Pantothensäure produzierenden Mikroorganismus erfindungsgemäß umfaßt.

Unter der Formulierung "Deregulation" ist erfindungsgemäß folgendes zu verstehen: Veränderung oder Modifikation mindestens eines Gens, das für ein Enzym in einem biosynthetischen Stoffwechselweg kodiert, so daß die Aktivität des Enzyms in dem Mikroorganismus verändert oder modifiziert ist. Bevorzugt ist, daß mindestens ein Gen, das für ein Enzym eines biosynthetischen Stoffwechselwegs kodiert, derart verändert ist, daß das Genprodukt verstärkt gebildet wird oder eine gesteigerte Aktivität aufweist. Der Begriff "deregulierter Stoffwechselweg" schließt auch einen biosynthetischen Stoffwechselweg mit ein, in den mehr als ein Gen, das für mehr als ein Enzym kodiert, so verändert oder modifiziert ist, daß die Aktivitäten von mehr als einem Enzym verändert oder modifiziert sind.
Veränderungen oder Modifikationen können beinhalten, sind aber nicht beschränkt auf: Entfernen des endogenen Promotors oder regulatorischer Elemente; Einfügen starker Promotoren, induzierbarer Promotoren oder mehrerer Promotoren gleichzeitig; Entfernen regulatorischer Sequenzen, so daß die Expression des Genprodukts verändert ist; Änderung der chromosomalen Lage des Gens; Veränderung der DNA-Sequenz in der Nähe des Gens oder innerhalb des Gens wie z. B. der ribosomalen Bindungsstelle (RBS); Erhöhung der Kopienzahl des Gens im Genom oder durch Einbringung von Plasmiden verschiedener Kopienzahl; Modifikation von Proteinen (z.B. regulatorischen Proteinen, Suppressoren, Enhancem, trankriptionellen Aktivatoren, und ähnliche), die bei der Transkription des Gens und/oder bei der Translation zum Genprodukt eine Rolle spielen. Dazu zählen auch alle anderen Möglichkeiten zur Deregulation der Expression von Genen die Stand der Technik sind wie z. B. die Verwendung von Antisense Oligonukleotiden, oder die Blockierung von Repressor Proteinen.
Deregulation kann auch Veränderungen in der kodierenden Region von Genen beinhalten, die z. B. zu Aufhebung von feedback Regulation in dem Genprodukt oder zu einer größeren oder kleineren spezifischen Aktivität des Genprodukts führen.

Darüber hinaus sind gentechnische Veränderungen an Enzymen erfindungsgemäß vorteilhaft, die den Abfluß von Vorstufen der Pantothensäure und/oder den Fluß der Pantothensäure zu Coenzym A beeinflussen. Beispielhaft für solche Enzyme kodierende Gene sind: alsD, avtA, ilvE, ansB, coaA, coaX und andere mehr. Diese Aufzählung dient der Erläuterung und ist keinesfalls limitierend für die vorliegende Erfindung.
Weiterhin sind gentechnische Veränderungen vorteilhaft, welche die zelluläre Bereitstellung von Cofaktoren (z. B. von Methylentetrahydrofolat, Redoxäquivalenten u. ä.) in für die Pantothensäure-Produktion optimaler Menge sichern.

In vorteilhafter Weise liegt so β-Alanin bereits in den Zellen in erhöhten Konzentrationen gegenüber entsprechend nicht-genetisch veränderten Mikroorganismen vor und muß somit nicht als Precursor dem Kulturmedium zugesetzt werden, wie es z. B. in EP-A-0 590 857 erforderlich ist. Vorteilhaft sind Mikroorganismen, deren Pantothensäure-(pan)- und/oder Isoleucin-Valin-(ilv)-Biosynthese und/oder Asparat-α-Decarboxylase (panD) dereguliert ist. Weiterhin ist eine zusätzliche Überexpression der Ketopanthoat-Reduktase (panE) in den Mikroorganismen von Vorteil.

Weiterhin erfindungsgemäß von Vorteil ist, wenn gegebenenfalls das coaA-Gen, das für die Synthese von CoenzymA erforderlich ist, in seiner Aktivität erniedrigt ist oder (beispielsweise in Bacillus-Arten) ganz ausgeschaltet ist. Bacillus enthält nämlich neben coaA ein weiteres Gen für diese enzymatische Funktion (= coaX). Auch die Aktivität dieses Gens coaX oder des korrespondierenden Enzyms kann verändert, bevorzugt erniedrigt, oder sogar deletiert werden, sofern coaA selbst noch eine ausreichende, wenn auch erniedrigte Enzymaktivität aufweist, d.h. die Enzymaktivität von coaA nicht ganz verloren gegangen ist. Neben der Überexpression der verschiedenen Gene ist auch eine genetische Manipulation der Promotorregionen dieser Gene in der Art vorteilhaft, daß diese Manipulation zu einer Überexpression der Genprodukte führt.

In einer Ausführungsvariante der vorliegenden Erfindung finden die gemäß der Anlage (WO 01/21772) beschriebenen Bakterienstämme, wie z.B. Bacillus subtilis PA 824 und/oder Derivate davon Einsatz. In einer weiteren Ausführungsvariante findet erfindungsgemäß der Mikroorganismus Bacillus subtilis PA 668, wie gemäß der Anlage (US 2004/0091979) beschrieben, Einsatz in dem erfindungsgemäßen Verfahren. Diese Stämme Bacillus subtilis PA824 und PA668 wurden wie folgt hergestellt:

Ausgehend von dem Stamm *Bacillus subtilis* 168 (Marburg Stamm ATCC 6051), der den Genotyp *trpC2* (Trp⁻) ausweist, wurde über Transduktion des Trp⁺ Markers (aus dem *Bacillus subtilis* Wildtyp W23) der Stamm PY79 erzeugt. In den Stamm PY79 wurden durch klassische gentechnische Methoden (wie z. B. in Harwood, C.R. and Cutting, S.M. (editors), Molecular Biological Methods for *Bacillus* (1990) John Wiley & Sons, Ltd., Chichester, England beschrieben) ΔpanB und ΔpanE1 Mutationen eingebracht.
Der resultierende Stamm wurde mit genomischer DNA des *Bacillus subtilis* Stammes PA221 (Genotyp P₂₆*panBCD, trpC2* (Trp⁻)) und genomischer DNA des *Bacillus subtilis* Stammes PA303 (Genotyp P₂₆*panE1*) transformiert. Der resultierende Stamm PA327 hat den Genotyp P₂₆*panBCD,* P₂₆*panE1* und ist Tryptophan auxotroph (Trp⁻). Mit dem *Bacillus subtilis* Stamm PA327 wurde in 10 ml Kulturen mit SVY-Medium (25 g/L Difco Veal Infusion Broth, 5 g/L Difco Yeast Extract, 5 g/L Na-Glutamat, 2,7 g/L Ammoniumsulfat auf 740 ml Wasser auffüllen, autoklavieren, anschließend Zugabe von 200 ml 1 M Kaliumphosphat, pH 7,0 und 60 ml 50% steriler Glucoselösung), das mit 5 g/L β-Alanin und 5 g/L α-Ketoisovalerat supplementiert war, Pantothensäure-Titer von bis zu 3,0 g/L (24 h) erreicht.

Die Herstellung des *Bacillus subtilis* Stammes PA221 (Genotyp P₂₆*panBCD, trpC2* (Trp⁻)) ist in folgendem Abschnitt beschrieben :
Durch klassische gentechnische Methoden wurde mit Hilfe der Sequenzinformation des *panBCD* Operons von *E. coli* (siehe Merkel et al., FEMS Microbiol. Lett., 143, 1996:247-252) ausgehend von einer *Bacillus subtilis* GP275 Plasmid-Bibliothek das *panBCD* Operon von *Bacillus* kloniert. Zur Klonierung wurde der *E. coli* Stamm BM4062 (bir^{ts}) und die Information, daß das *Bacillus* Operon in der Nähe des *birA* Gens liegt, verwendet. Das *panBCD* Operon wurde in ein in *E. coli* replizierbares Plasmid eingebracht. Zur Verbesserung der Expression des *panBCD* Operons wurden starke, konstitutive Promotoren des *Bacillus subtilis* Phagen SP01 (P₂₆) verwendet und die Ribosomenbindungsstelle (=RBS) vor dem *panB-*Gen durch eine artifizielle RBS ersetzt. Vor die P₂₆*panBCD* Kassette auf dem Plasmid wurde ein DNA Fragment, das unmittelbar *upstream* des nativen *panB* Gens in *Bacillus* liegt, ligiert. Dieses Plasmid wurde in den *Bacillus subtilis* Stamm RL-1 (durch klassische Mutagenese erhaltenes Derivat des *Bacillus subtilis* 168 (Marburg Stamm ATCC 6051), Genotyp *trpC2* (Trp⁻)) transformiert und durch homologe Rekombination wurde das native *panBCD* Operon durch das p₂₆panBCD Operon ersetzt. Der resultierende Stamm heißt PA221 und hat den Genotyp P₂₆*panBCD, trpC2* (Trp⁻).
Mit dem *Bacillus subtilis* Stamm PA221 wurde in 10 ml Kulturen mit SVY-Medium, das mit 5 g/L β-Alanin und 5 g/L α-Ketoisovalerat supplementiert war, Pantothensäure-Titer von bis zu 0,92 g/L (24 h) erreicht.

Die Herstellung des *Bacillus subtilis* Stamms PA303 (Genotyp P₂₆*panE1*) ist in folgendem Abschnitt beschrieben :
Mit Hilfe der *E. coli panE* Gensequenz wurde die *Bacillus panE* Sequenz analog kloniert. Es zeigte sich, daß in *B. subtilis* zwei Homologe des *panE* Gens von *E. coli* existieren, die mit *panE1* und *panE2* bezeichnet wurden. Durch Deletionsanalysen zeigte sich, daß das *panE1* Gen für 90 % der Pantothensäure Produktion verantwortlich ist, während die Deletion des *panE2* Gens keinen signifikanten Effekt auf die Pantothensäure Produktion hatte. Auch hier wurde analog zur Klonierung des *panBCD* Operons der Promotor durch den starken konstitutiven Promotor P₂₆ ersetzt und die Ribosomenbindungsstelle vor dem *panE1* Gen durch die artifizielle Bindungsstelle ersetzt. Das P₂₆*panE1* Fragment wurde in einen Vektor kloniert, des so gestaltet war, daß das P₂₆*panE1* Fragment in den original *panE1* locus im Genom von *Bacillus subtilis* integrieren konnte. Der nach Transformation und homologer Rekombination resultierende Stamm heißt PA303 und hat den Genotyp P₂₆*panE1*.

Mit dem *Bacillus subtilis* Stamm PA303 wurde in 10 ml Kulturen mit SVY-Medium, das mit 5 g/L β-Alanin und 5 g/L α-Ketoisovalerat supplementiert war, Pantothensäure-Titer von bis zu 1,66 g/L (24 h) erreicht.

Die weitere Stammkonstruktion erfolgte durch Transformation von PA327 mit einem Plasmid, welches das P₂₆*ilvBNC* Operon und das Marker-Gen für Spectinomycin enthielt. Das P₂₆*ilvBNC* Operon integrierte in den *amyE* locus, was durch PCR nachgewiesen wurde. Ein Transformante wurde als PA340. (Genotyp P₂₆*panBCD,* P₂₆*panE1,* P₂₆*ilvBNC,* specR, *trpC2* (Trp⁻)) bezeichnet.
Mit dem *Bacillus subtilis* Stamm PA340 wurde in 10 ml Kulturen mit SVY-Medium, das nur mit 5 g/L β-Alanin supplementiert war, Pantothensäure-Titer von bis zu 3,6 g/L (24 h) erreicht, in 10 ml Kulturen mit SVY-Medium, das mit 5 g/L β-Alanin und 5 g/L α-Ketoisovalerat supplementiert war, wurden Pantothensäure-Titer von bis zu 4,1 g/L (24 h) erreicht.

Des weiteren wurde in den Stamm PA340 eine deregulierte *ilvD*-Kassette eingebracht. Dazu wurde ein Plasmid, welches das *ilvD* Gen unter der Kontrolle des P₂₆ Promotors mit der artifiziellen RBS2 enthält, in PA340 transformiert. Dabei wurde das P₂₆*ilvD* Gen durch homologe Rekombination in den original *ilvD* locus integriert. Der resultierende Stamm PA374 hat den Genotyp P₂₆*panBCD,* P₂₆*panE1,* P₂₆*ilvBNC,* P₂₆*ilvD,* specR und *trpC2* (Trp⁻).
Mit dem *Bacillus subtilis* Stamm PA374 wurde in 10 ml Kulturen mit SVY-Medium, das nur mit 5 g/L β-Alanin supplementiert war, Pantothensäure-Titer von bis zu 2,99 g/L (24 h) erreicht.

Um mit dem Stamm PA374 β-Alanin zufütterungsfrei Pantothensäure zu produzieren, wurden zusätzliche Kopien des für die Aspartat-α-decarboxylase kodierenden Gens *panD* in den Stamm PA374 eingebracht. Dazu wurde chromosomale DNA des Stammes PA401 in PA374 transformiert. Durch Selektion auf Tetrazyklin wurde der Stamm PA377 erhalten.
Der resultierende Stamm PA377 hat den Genotyp P₂₆*panBCD,* P₂₆*panE1,* P₂₆*ilvBNC,* P₂₆*ilvD*, specR, tetR und *trpC2* (Trp⁻).
Mit dem *Bacillus subtilis* Stamm PA377 wurde in 10 ml Kulturen mit SVY-Medium Vorstufen-zufütterungsfrei Pantothensäure-Titer von bis zu 1,31 g/L (24 h) erreicht.

Die Herstellung des *Bacillus subtilis* Stamms PA401 (Genotyp P₂₆*panD*) ist in folgendem Abschnitt beschrieben :
Das Bacillus subtilis *panD* Gen wurde aus dem *panBCD* Operon in einen Vektor, der das Tetrazyklin Marker Gen trägt, kloniert. Vor das *panD* Gen wurde der Promotor P₂₆ und eine oben beschriebene artifizielle RBS kloniert. Durch Restriktionsverdau wurde ein Fragment, das das Tetrazyklin Marker Gen und das P₂₆*panD* Gen enthielt, hergestellt. Dieses Fragment wurde religiert und in den oben beschriebenen Stamm PA221 transformiert. Dabei integrierte das Fragment in das Genom des Stamms PA211. Der resultierende Stamm PA401 hat den Genotyp P₂₆*panBCD,* P₂₆*panD,* tetR und *trpC2* (Trp⁻).
Mit dem *Bacillus subtilis* Stamm PA401 wurde in 10 ml Kulturen in SVY-Medium, das mit 5 g/L α-Ketoisovalerat supplementiert war, Pantothensäure-Titer von bis zu 0,3 g/L (24 h) erreicht. In 10 ml Kulturen mit SVY-Medium das mit 5 g/L D-Pantoinsäure und 10 g/L L-Aspartat supplementiert war, wurden Pantothensäure-Titer von bis zu 2,2 g/L (24 h) erreicht.

Ausgehend von Stamm PA377 wurde durch Transformation mit chromosomaler DNA von Stamm PY79 ein Tryptophan prototropher Stamm generiert. Dieser Stamm PA824 hat den Genotyp P₂₆*panBCD,* P₂₆*panE1,* P₂₆*ilvBNC*, P²⁶*ilvD,* specR, tetR und Trp⁺.
Mit dem *Bacillus subtilis* Stamm PA824 wurde in 10 ml Kulturen in SVY-Medium Vorstufen-zufütterungsfrei Pantothensäure-Titer von bis zu 4,9 g/L (48 h) (Vergleich PA377: bis zu 3,6 g/L in 48 h) erreicht. Die genaue Konstruktion der Stämme ist gemäß der Anlage der WO 01/21772 zu entnehmen.

Die Herstellung von PA668 ist in folgendem Abschnitt beschrieben:
Das *Bacillus panB* Gen wurde aus dem Wild-Typ *panBCD* Operon kloniert und in einen Vektor insertiert, der neben einem Chloramphenicol-Resistenz-Gen auch *B. subtilis* Sequenzen des *vpr locus* enthält.
   Der starke konstitutive Promotor P₂₆ wurde vor das 5' Ende des *panB* Gens eingeführt. Ein Fragment, das das P₂₆*panB* Gen, das Marker-Gen für Chloramphenicol-Resistenz sowie *Bacillus subtilis vpr* Sequenzen enthält wurde durch Restriktionsverdau erhalten. Das isolierte Fragment wurde religiert und der Stamm PA824 damit transformiert. Der erhaltene Stamm wurde mit PA668 bezeichnet. Der Genotyp von PA668 ist: P₂₆*panBCD,* P₂₆*panE1,* P₂₆*ilvBNC,* P₂₆*ilvD,* P₂₆*panB,* specR, tetR, CmR and Trp⁺.
   Zwei Kolonien von PA668 wurde isoliert und mit PA668-2A, die andere mit PA668-24 bezeichnet.
   Mit *B. subtilis* Stamm PA668-2A werden in 10mL Kulturen in SVY-Medium ohne Zufütterung von Vorstufen Pantothensäure-Titer von 1,5 g/L in 48 h erreicht. In 10 mL Kulturen, die mit 10g/L Aspartat supplementiert sind, werden Titer bis zu 5 g/L erreicht.
   Mit *B. subtilis* Stamm PA668-24 werden in 10mL Kulturen in SVY-Medium ohne Zufütterung von Vorstufen Pantothensäure-Titer von 1,8 g/L in 48 h erreicht. In 10 mL Kulturen, die mit 10g/L L-Aspartat supplementiert sind, werden Titer bis zu 4,9 g/L erreicht.

Die genaue Konstruktion der Stämme ist gemäß den Anlagen der WO 01/21772 und US 2004/0091979 zu entnehmen.

Mit dem oben beschriebenen Stamm PA377 werden bei Glucose-limitierter Fermentation in SVY-Medium (25 g/L Difco Veal Infusion Broth, 5 g/L Difco Yeast Extract, 5 g/L Tryptophan, 5 g/L Na-Glutamat, 2 g/L (NH₄)₂SO₄, 10 g/L KH₂PO₄, 20 g/L K₂HPO₄, 0,1 g/L CaCl₂, 1 g/L MgSO₄, 1 g/L Natriumcitrat, 0,01 g/L FeSO₄*7 H₂O und 1 ml/L einer Spurensalzlösung folgender Zusammensetzung : 0,15 g Na₂MoO₄ x 2 H₂O, 2,5 g H₃BO₃, 0,7 g CoCl₂ x 6 H₂O, 0,25 g CuSO₄ x 5 H₂O, 1,6 g MnCl₂ x 4 H₂O, 0,3 g ZnSO₄ x 7 H₂O, mit Wasser auf 1 1 aufgefüllt)) im 10 L Maßstab bei kontinuierlicher Zufütterung einer Glucose-Lösung in 36 h (48 h) Pantothensäure-Konzentrationen in der Fermentationsbrühe von 18-19 g/L (22-25 g/L) erreicht.

Bei Glucose-limitierter Fermentation von PA824, dem Tryptophan-prototrophen Derivat von PA377, in Hefeextrakt-Medium (10 g/L Difco Yeast Extract, 5 g/L Na-Glutamat, 8 g/L (NH₄)₂SO₄, 10 g/L KH₂PO₄, 20 g/L K₂HPO₄, 0,1 g/L CaCl₂, 1 g/L MgSO₄, 1 g/L Natriumcitrat, 0,01 g/L FeSO₄*7 H₂O und 1 ml/L der oben beschriebenen Spurensalzlösung) werden im 10 L Maßstab bei kontinuierlicher Zufütterung einer Glucose-Lösung in 36 h, 48 h und 72 h folgende Pantothensäure-Konzentrationen in Fermentationsbrühen erreicht: 20 g/L, 28 g/L und 36 g/L.

Durch weitere Medienoptimierung wird mit Stamm PA824 bei Glucose-limitierter Fermentation in einem Medium bestehend aus 10 g/L Difco Yeast Extract, 10 g/L NZ Amine A (Quest International GmbH, Erftstadt), 10 g/L Na-Glutamat, 4 g/L (NH₄)₂SO₄, 10 g/L KH₂PO₄, 20 g/L K₂HPO₄, 0,1 g/L CaCl₂, 1 g/L MgSO₄, 1 g/L Natriumcitrat, 0,01 g/L FeSO₄*7 H₂O und 1 ml/L der oben beschriebenen Spurensalzlösung im 10 L Maßstab bei kontinuierlicher Zufütterung einer Glucose-Lösung in 36h (48h) Pantothensäure-Konzentrationen von 37 g/L (48 g/L) in Fermentationsbrühen erreicht.
Weitere Erhöhungen der Pantothensäure-Konzentration in der Fermentationsbrühe sind durch weitere Medienoptimierung, durch Erhöhung der Fermentationsdauer, durch Verfahrens- und Stammverbesserung sowie durch Kombinationen der einzelnen Schritte denkbar. So sind die oben beschriebenen Pantothensäure-Konzentrationen auch durch Fermentation von Stämmen zu erreichen, die Derivate des oben beschriebenen PA824 sind. Derivate können durch klassische Stammentwicklung sowie durch weitere gentechnische Manipulationen hergestellt werden. Durch Medien-, Stamm- und Fermentationsverfahrensentwicklung können die Pantothensäure-Titer in den Fermentationsbrühen auf über 40, 45, 50, 55, 60, 65, 70, 75, 80, 85, und > 90 g/L gesteigert werden.

Ein wesentlicher Vorteil des erfindungsgemäßen Verfahrens ist, daß die Fermentation in einem Kulturmedium durchgeführt wird, das außer wenigstens einer Kohlenstoff- und Stickstoffquelle als Ausgangsverbindungen keine weiteren Vorstufen (Precursor) enthält. D.h. die Biosynthese von D-Pantothensäure ist von der Zufütterung weiterer Vorstufen unabhängig. Unter solchen Vorstufen sind erfindungsgemäß Substanzen wie z.B. β-Alanin und/oder L-Aspartat und/oder L-Valin und/oder α-Ketoisovalerat und/oder deren Kombinationen zu verstehen.
In einer bevorzugten Variante des erfindungsgemäßen Verfahren wird die Fermentation des D-Pantothensäure produzierenden Mikroorganismus in einem Kulturmedium durchgeführt, welches eine Kohlenstoff- und eine Stickstoffquelle enthält, dem aber kein freies β-Alanin und/oder β-Alanin-Salze zugesetzt ist oder im Verlauf der Fermentation zugeführt wird. D. h. zur Herstellung von D-Pantothensäure in Bereichen von wenigstens 10 g/l Kulturmedium, bevorzugt von wenigstens 20 g/l, besonders bevorzugt von wenigstens 40 g/l, höchst besonders bevorzugt von wenigstens 60 g/l und insbesondere von wenigstens 70 g/l, ist erfindungsgemäß keine Zufütterung von freiem β-Alanin und/oder β-Alanin-Salzen erforderlich.
Die Unabhängigkeit von der Zufütterung von Vorstufen stellt, insbesondere einen wesentlichen wirtschaftlichen Vorteil des erfindungsgemäßen Verfahrens gegenüber bekannten Verfahren dar, da eine Vielzahl von Vorstufen sehr teuer sind.

Die Zugabe von β-Alanin und/oder β-Alanin-Salzen ist erfindungsgemäß jedoch nicht ausgeschlossen, so daß folglich die Ausbeute an D-Pantothensäure durch die Zugabe von β-Alanin und/oder β-Alanin-Salzen noch weiter verbessert werden kann. Geht man beispielsweise davon aus, daß alle erforderlichen Vorstufen der Pantothensäure in ausreichender Menge vorhanden sind lediglich die Aktivität des panD-Gens eine weitere Steigerung der Pantothensäure-Produktion limitiert, so kann die Ausbeute an Pantothensäure z.B. um weitere 50% durch die Zugabe von freiem β-Alanin und/oder β-Alanin-Salzen gesteigert werden.
In einer vorteilhaften Variante der vorliegenden Erfindung können bis zu 20 g/l an freiem β-Alanin und/oder β-Alanin-Salzen dem Kulturmedium zur zusätzlichen Steigerung der Pantothensäureausbeute um mehr als 50 % zugesetzt werden. Bevorzugt ist der Zusatz von etwa 15 g/l an freiem β-Alanin und/oder β-Alanin-Salzen zum Kulturmedium.

Beispiele erfindungsgemäß geeigneter Kohlenstoffquellen zum Einsatz in einem Kulturmedium zur Fermentation der zuvor genannten Mikroorganismen sind Zucker, wie Stärkehydrolysate (Mono-, Di-, Oligosaccharide), bevorzugt Glucose oder Saccharose sowie Rüben- oder Rohrzuckermelasse, Proteine, Proteinhydrolysate, Sojamehl, Maisquellwasser, Fette, freie Fettsäuren, rückgeführte Zellen aus bereits durchgeführten Fermentationen oder deren Hydrolysate sowie Hefeextrakt. Diese Aufzählungen sind nicht limitierend für die vorliegende Erfindung.

Ferner zeichnet sich das vorliegende Verfahren in vorteilhafter Weise dadurch aus, daß der Gesamt-Zuckergehalt bis zum Ende der Fermentation auf ein Minimum reduziert wird, da dieser anderenfalls die spätere Trocknung und/oder Formulierung der Fermentationslösung durch Verkleben erschwert. Dies kann erfindungsgemäß dadurch erreicht werden, daß die Fermentation noch einige Zeit weitergeführt wird, nachdem die Kohlenstoffquelle verbraucht ist (bei Kultivierung im batch-Betrieb) oder nachdem die Kohlenstoffzufuhr (bei einer Prozeßführung im fed-batch oder repeated-fed-batch-Betrieb) unterbrochen ist und/oder derart reguliert ist, daß die Konzentration der Kohlenstoffquelle nahezu Null ist (bei fed-batch, repeated-fed-batch oder kontinuierlicher Prozeßführung).
Dies erfolgt erfindungsgemäß dadurch, daß nach Unterbrechung der Zudosierung der Kohlenstoffquelle (z. B. Zuckerlösung) die Fermentation bis zum Erreichen der Gelöstsauerstoffkonzentration (pO₂) auf wenigstens 80 %, bevorzugt 90 % und besonders bevorzugt 95 % des Sättigungswertes in der Fermentationslösung weitergeführt wird.

Beispiele erfindungsgemäß geeigneter Stickstoffquellen sind Ammoniak, Ammoniumsulfat, Harnstoff, Proteine, Proteinhydrolysate oder Hefeextrakt. Auch diese Aufzählung ist nicht limitierend für die vorliegende Erfindung.

Ferner enthält das Fermentationsmedium Mineralsalze und/oder Spurenelemente, wie Aminosäuren und Vitamine. Die genauen Zusammensetzungen geeigneter Fermentationsmedien sind zahlreich bekannt und dem Fachmann zugänglich.
Nach Inokkulation des Fermentationsmediums mit einem geeigneten D-Pantothensäure produzierenden Mikroorganismus (mit dem Fachmann bekannten Zelldichten) erfolgt, ggf. unter Zusatz eines Antischaummittels, die Kultivierung des Mikroorganismus. Eine ggf. erforderliche Regulierung des pH-Wertes des Mediums kann mit verschiedenen anorganischen oder organischen Laugen oder Säuren erfolgen, wie z.B. NaOH, KOH, Ammoniak, Phosphorsäure, Schwefelsäure, Salzsäure, Ameisensäure, Bersteinsäure, Zitronensäure o.ä..

Aufgrund der während der Fermentation eingesetzten Puffersysteme, die wie zuvor beschrieben, z.B. NaOH, KOH, Ammoniak, Phosphorsäure, Schwefelsäure, Salzsäure, Ameisensäure, Bersteinsäure, Zitronensäure o.ä. sein können, liegt die gebildete D-Pantothensäure in der Fermentationslösung je nach eingesetztem Puffersystem in Form des/der jeweiligen Salze(s) vor. Da hierbei insbesondere die Salze der D-Pantothensäure in Form ihrer einwertigen Kationen unvorteilhaft sind, wird die Fermentationslösung erfindungsgemäß durch Nanofiltration aufbereitet. Hierzu werden zunächst dem gebildeten D-Pantothenat erfindungsgemäß Salze enthaltend mehrwertige Kationen zugeführt, wodurch mehrwertige Salze der D-Pantothensäure ausgebildet werden. Erfindungsgemäß kann die Zugabe von Salzen enthaltend mehrwertige Kationen in fester Form oder wässriger Lösung während, bevorzugt am Ende, oder nach der Fermentation in Schritt a) erfolgen. Die Zuführung einer wässrigen Lösung enthaltend mehrwertige Kationen kann beispielsweise kontinuierlich erfolgen.

Ferner kann in einem der Nanofiltration vorgeschalteten Schritt, also vor der Nanofiltration in Schritt c) des erfindungsgemäßen Verfahrens, eine Abtrennung von Zellmasse oder von in der Lösung ausgefallenen Komponenten erfolgen. Hierbei kann die Abtrennung durch Dekantieren oder Membranfiltration, bevorzugt Ultrafiltration erfolgen. In einer Variante des erfindungsgemäßen Verfahrens wird die Membranfiltration als Diafiltration durchgeführt. Auch hier kann erfindungsgemäß die Zugabe von Salzen enthaltend mehrwertige Kationen in fester Form oder wässriger Lösung während oder nach einer Membranfiltration einer D-Pantothenat-haltigen Lösung erfolgen. Beispielsweise erfolgt die Zuführung einer wässrigen Lösung enthaltend mehrwertige Kationen dabei kontinuierlich.

Bei der Abtrennung von Zellmasse und/oder in der Lösung ausgefallene Komponenten, wie z. B. schwerlösliche oder unlösliche Phospatsalze bzw. Sulfatsalze, Enzyme, Hormone, Proteine, Antibiotika, Pyrogene, Viren, Polysaccharide, Kolloide, Tenside, Pestizide oder andere organische Substanzen, beruht die Trennung auf der Ausnutzung von Schwerkraft, Zentrifugalkraft, Druck oder Vakuum. Beispielhafte Verfahren sind u.a.: Dekantieren, Elutriation, Sieben, Windschichten, Sortieren, Filtration, Dialyse, Sedimentation, Mikrofiltration, Ultrafiltration, Flotation, Schaumfraktionierung, Sink-Schwimm-Aufbereitung, Klären, Zentrifugieren oder Abscheiden. Als Menibranfiltration faßt man durch eine Druckdifferenz zwischen Feed- und Permeatseite betriebene Membran-Trennverfahren, wie Mikrofiltration oder Ultrafiltration zusammen. Die Verfahren unterscheiden sich z. B. durch ihre Trenngrenzen. So wird bei der Ultrafiltration die Ausschlußgrenze (cut-off) nicht wie z.B. bei der Mikrofiltration auf die Partikelgröße bezogen, sondern auf die Molmasse, die im Bereich von etwa 10³ bis 2x10⁶ Da liegt. Bei der Ultrafiltration fällt neben dem Filtrat (Permeat) das sogenannte Konzentrat (Retentat) an.

Zur Abtrennung fester oder An- oder Abreicherung gelöster mittel- und hochmolekularer Stoffe werden vorteilhaft asymmetrisch strukturierte, poröse Membranen eingesetzt.
Die erfindungsgemäß eingesetzten Membranen können in einer vorteilhaften Variante aus einer Trennschicht, welche die eigentliche Stofftrennung bewirkt, und einer ein- oder mehrlagigen Stützschicht, welche die Trennschicht trägt und gröbere Poren als die Trennschicht aufweist, aufgebaut sein. Die Trennschichten sowie die Stützschicht können aus organischen oder anorganischen Polymeren, Keramik, Metall oder Kohlenstoff bestehen und müssen in dem Reaktionsmedium und bei der Prozesstemperatur stabil sein. Beispiele hierfür sind in Tabelle 1 aufgelistet, jedoch nicht limitierend für die vorliegende Erfindung:

Die Membranen können in Form von Schläuchen, Rohren, Kapillaren, Hohlfasern oder Flachmembranen in an sich bekannten Flach-, Rohr-, Multikanalelement-, Kapillar- oder Wickelmodulen eingesetzt werden.
Die optimalen transmembranen Drücke zwischen Retentat und Permeat liegen im wesentlichen, abhängig vom Durchmesser der Membranporen bzw. der Trenngrenze (angegeben in Molekulargewichtseinheiten), der mechanischen Stabilität der Membran und je nach Membran-Art zwischen 1 und 40 bar. Höhere transmembrane Drücke führen in der Regel zu höheren Permeatflüssen. Dabei kann in dem Fall in dem der Feed (aufzubereitende Lösung) mit einem zu hohen Druck zugeführt wird, der transmembrane Druck durch Anheben des Permeatdruckes angepaßt werden.

Die Betriebstemperatur ist abhängig von der Produkt- und der Membranstabilität. Sie liegt zwischen etwa 20 und 90 °C, bevorzugt zwischen etwa 40 und 70 °C. Höhere Temperaturen führen zu höheren Permeatflüssen. Dabei sind z.B. Membranen gemäß Tabelle 2 einsetzbar, die jedoch nicht limitierend für die vorliegende Erfindung sind.

Die Zellabtrennung kann erfindungsgemäß in vorteilhafter Weise auch durch eine Sonderform der Membranfiltration, nämlich eine Diafiltration erfolgen.
Die Diafiltration kann diskontinuierlich erfolgen, indem die Lösung enthaltend mehrwertige Salze der D-Pantothensäure über einen Kreislauf, enthaltend einen Behälter, eine Pumpe und ein oder mehrere Membranmodule, geführt wird und dabei die Drücke in den Membranmodulen so eingestellt werden, daß Permeat anfällt. Dabei wird stetig oder zu bestimmten Zeiten Wasser oder eine wässrige Lösung zugegeben, die das abzutrennende Produkt nicht oder in geringerer Konzentration enthält, als zum Zeitpunkt der Zugabe in den Trennkreislauf. Die wässrige Lösung kann erfindungsgemäß Salze mehrwertiger Kationen, wie z. B. Calcium- oder Magnesium-Halogenide oder deren Kombinationen, bevorzugt Calcium- und/oder Magnesium-Chlorid, enthalten.
Die Zellabtrennung mittels Diafiltration kann erfindungsgemäß auch kontinuierlich erfolgen, wobei vorzugsweise mehrere Membranmodule in Reihe geschaltet sind oder jeweils ein oder mehrere Membranmodul-enthaltende Pumpkreisläufe in Reihe geschaltet sind. Vor, zwischen oder nach den Membranmodulen oder Pumpkreisläufen kann Wasser oder eine wässrige Lösung, die das abzutrennende Produkt nicht oder in geringerer Konzentration enthält als am Ort der Zugabe, zugegeben werden, wobei, wie bei der diskontinuierlichen Variante, die wässrige Lösung Salze mehrwertiger Kationen, wie z. B. Calcium- oder Magnesium-Halogenide oder deren Kombinationen, bevorzugt Calcium- und/oder Magnesium-Chlorid, enthalten kann.
Erfindungsgemäß kann die Ultrafiltration oder Diafiltration direkt mit dem Fermentationsaustrag durchgeführt werden oder nach einer Behandlung des Fermentationsaustrages, z.B. durch Zentrifugation, Dekantierung oder ähnliches Vorgehen, erfolgen.

Sofern erfindungsgemäß eine Abtrennung von Zellmasse oder von in der Lösung ausgefallenen Komponenten durchgeführt wird, kann die Zugabe von Salzen enthaltend mehrwertige Kationen gemäß Schritt b) des erfindungsgemäßen Verfahrens vor, während oder nach der Ultrafiltration oder Diafiltration erfolgen. In Varianten des erfindungsgemäßen Verfahrens werden als mehrwertige Kationen beispielsweise Calcium- und/oder Magnesium-Chlorid, -Nitrat, -Hydroxid, -Formiat, - Acetat, -Propionat, -Glycinat und/oder -Lactat zugesetzt. Hierbei kann als mehrwertiges Kation beispielsweise Ca²⁺ in einer Konzentration von 0,05 - 50 mol Ca²⁺/mol D-Pantothenat, bevorzugt 0,2 - 2 mol Ca²⁺/mol D-Pantothenat zugeführt werden.

In Schritt c) des erfindungsgemäßen Verfahrens wird sodann die Lösung enthaltend mehrwertige Salze der D-Pantothensäure durch Nanofiltration aufbereitet, wobei die mehrwertigen Salze der D-Pantothensäure angereichert und gleichzeitig unerwünschte einwertige lonen, bevorzugt einwertige Kationen, wie, z. B. Ammonium-, Natrium- oder Kalium-lonen, abgereichert werden. Das erfindungsgemäße Verfahren zeichnet sich dadurch aus, daß der Gehalt an einwertigen Kationen, bevorzugt Ammonium-, Kalium- und/oder Natrium-Ionen, auf eine Konzentration von ≤ 5 g/kg Lösung reduziert wird.

Die vorliegende Erfindung umfaßt alle gewerblich verfügbaren Nanofiltrationssysteme. Die Trennung erfolgt vorteilhaft an asymmetrisch strukturierten, porösen Membranen. In einer bevorzugten Variante des vorliegenden Verfahrens werden dazu Membranen eingesetzt, die aus einer Trennschicht, welche die eigentliche Stofftrennung bewirkt, und einer ein- oder mehrlagigen Stützschicht, welche die Trennschicht trägt und gröbere Poren als die Trennschicht aufweist, aufgebaut sind. Die Trennschichten sowie die Stützschicht können aus organischen Polymeren, Keramik, Metall oder Kohlenstoff bestehen und müssen in dem Reaktionsmedium und bei der Prozesstemperatur stabil sein. Bevorzugte Materialien für die Trennschicht sind Polyamide, Polyimide oder Polypiperazine. Die Trennschichten können auch eine positive oder negative Oberflächenladung aufweisen. Als Beispiel für eine anionisch funktionalisierte Nanofiltrationsmembran ist die Membran DESAL 5 DK zu nennen, die jedoch die vorliegende Erfindung nicht auf die ausschließliche Verwendung dieser Membran limitiert.

Die Membranen können in Form von Schläuchen, Kapillaren, Hohlfasern oder Flachmembranen und in an sich bekannten Flach-, Rohr-, Multikanalelement-, Kapillar- oder Wickelmodulen eingesetzt werden.

In vorteilhaften Varianten des erfindungsgemäßen Verfahrens wird bei der Nanofiltration in Schritt c) eine Druckdifferenz über der Membran im Bereich von 5-100 bar, bevorzugt 20-80 bar und besonders bevorzugt bei 40-70 bar aufgebaut.
Die Prozeßtemperatur liegt vorteilhafter Weise zwischen 20 und 80, bevorzugt zwischen 30 und 60 °C. Ferner kann die Nanofiltration in einer dem Fachmann bekannten Weise in einem oder mehreren Schritten kontinuierlich oder diskontinuierlich betrieben werden.

In einer bevorzugten Variante wird jeweils vor einem oder mehreren Nanofiltrationsschritt(en) ein Salz enthaltend mehrwertige Kationen in fester Form oder in wässriger Lösung zugegeben. Erfindungsgemäß werden die mehrwertigen Kationen als Calcium- und/oder Magnesium-Chlorid, -Nitrat, -Hydroxid, -Formiat, - Acetat, -Propionat, -Glycinat und/oder -Lactat zugesetzt. Hierbei kann als mehrwertiges Kation Ca²⁺ in einer Konzentration von 0,05 - 50 mol Ca²⁺/mol D-Pantothenat, bevorzugt 0,2 - 2 mol Ca²⁺/mol D-Pantothenat (bezogen auf den Zustand nach der Zumischung) zugeführt werden.
Das erfindungsgemäße Verfahren zeichnet sich dadurch aus, daß die Zugabe von Salzen enthaltend mehrwertige Kationen in fester Form oder wässriger Lösung während, bevorzugt am Ende, oder nach der Fermentation in Schritt a) oder während oder nach der Zellabtrennung erfolgt.
Erfindungsgemäß vorteilhaft kann außerdem die Zugabe von Salzen enthaltend mehrwertige Kationen während des Nanofiltrationsschrittes erfolgen. Ferner kann die Zuführung einer wässrigen Lösung enthaltend mehrwertige Kationen kontinuierlich erfolgen.

In einer weiteren Variante des vorliegenden Verfahrens ist es denkbar, daß bei einem oder mehreren der Nanofiltration vorgelagerten Verfahrensschritten Lösungen mit unterschiedlicher Produktkonzentration anfallen. Diese genannten Lösungen können durch eine Nanofiltration in der Weise weiter verarbeitet werden, dass die genannten Lösungen in der Reihenfolge aufsteigender Produktkonzentrationen in aufeinanderfolgenden Nanofiltrationsschritten zugeführt werden.

Erfindungsgemäß werden durch das zuvor beschriebene erfindungsgemäße Verfahren im Retentat der Nanofiltration vor allem die mehrwertigen Salze der Pantothensäure angereichert. In der Permeatlösung werden hauptsächlich die monovalenten Ionen, bei Einsatz einer anionisch funktionalisierten Nanofiltrationsmembran die einwertigen Kationen, angereichert. Der Gehalt an einwertigen Kationen, bevorzugt Ammonium-, Kalium- und/oder Natrium-lonen, im Retentat kann dabei auf eine Konzentration von ≤ 5 g/kg Lösung reduziert werden.
Erfindungsgemäß kann das Permeat der Nanofiltration oder ein Teil davon in die Fermentation in Schritt a) des erfindungsgemäßen Verfahrens zurückgeführt werden. Diese Rückführung des Permeats oder Teile davon kann kontinuierlich erfolgen. Die zuvor beschriebenen zusätzlich zur Nanofiltration durchgeführten Verfahrensschritte dienen der Vorkonzentrierung oder weiteren Aufkonzentrierung von D-Pantothenats in Form mehrwertiger Salze.

Ein weiterer Vorteil der erfindungsgemäß eingesetzten Nanofiltration ist, daß die Reduktion der monovalenten Kationen (in der Retentatlösung) gleichzeitig mit einer Volumenverringerung des Retentats einhergehen kann. Die Aufarbeitung der D-Pantothenat-haltigen Fermentationslösung über Nanofiltration kann somit erfindungsgemäß als lonenaustausch- und Konzentrierungsverfahren zur Herstellung von D-Pantothenat eingesetzt werden.
Dies führt in vorteilhafter Weise zu einer Vereinfachung und gleichzeitig gesteigerten Effizienz der nachfolgenden Verfahrensschritte. Beispielsweise kann der Energieaufwand in der Trocknung aufgrund der Aufkonzentrierung wesentlich reduziert werden.

In einer bevorzugten Ausführungsform der vorliegenden Erfindung wird die Fermentationslösung durch Zentrifugieren und/oder Dekantieren und/oder Ultrafiltration von der Zellmasse befreit. Nach Zusatz von 0,05 bis 50 mol (Ca²⁺)-Ionen/mol Pantothenat-Ion, bevorzugt 0,2 - 2 mol Ca²⁺-lonen/mol Pantothenat-Ion, die bevorzugt in Form einer verdünnten Lösung mit 0,01 - 10 mol Ca²⁺/l vorgelegt werden, wird die so erhaltene Lösung in ein Nanofiltrationsmodul eingeleitet. Die Druckdifferenz über der Membran liegt im Bereich von etwa 5 - 100 bar, bevorzugt etwa 20 - 80 bar, besonders bevorzugt etwa 40-70 bar. Vor oder während der Nanofiltration kann dabei eine Ca²⁺-Ionen enthaltende wässrige Lösung zu der feedseitig die Membran überströmenden Lösung zugegeben werden. Das Retentat besitzt ein Volumen von 30 - 200% der Ausgangslösung. Ferner werden etwa 5 - 99%, bevorzugt 30 - 80 % der enthaltenen monovalenten Kationen entfernt.

Das Retentat enthaltend vorzugsweise Calcium-D-Pantothenat, Magnesium-D-Pantothenat oder eine Mischung davon, wird anschließend einer Trocknung und/oder Formulierung unterzogen. Die Trocknung und/oder Formulierung der Calcium- und/oder Magnesium-D-Pantothenat-enthaltende Lösung erfolgt nach an sich bekannten Verfahren, wie beispielsweise Sprühtrocknung, Sprühgranulation, Wirbelschichttrocknung, Wirbelschichtgranulation, Trommeltrocknung oder Spin-flash Trocknung (Ullmann's Encyclopedia of Industrial Chemistry, 6^{th} edition, 1999, electronic release, Kapitel "Drying of Solid Materials"). Die Gaseintrittstemperatur bei Konvektionstrocknung liegt im Bereich von 100 - 280 °C, bevorzugt bei 120 - 210 °C. Die Gasaustrittstemperatur liegt bei 50 - 180 °C, bevorzugt bei 60 - 150 °C. Zur Einstellung einer gewünschten Partikelgrößenverteilung und der damit verbundenen Produkteigenschaften können Feinpartikel abgetrennt und zurückgeführt werden. Ferner kann Grobgut in einer Mühle gemahlen und ebenfalls anschließend zurückgeführt werden.

Das erfindungsgemäße Verfahren weist die Vorteile auf, daß unerwünschte Kationen effizient und nahezu vollständig entfernt werden und gleichzeitig eine Volumenreduktion erfolgt, welche die nachfolgenden Verfahrensschritte, insbesondere die Trocknung und/oder Formulierung vereinfacht oder effizienter macht. Ferner findet keine oder nur eine äußerst geringe Produktzersetzung statt, bei gleichzeitig hoher Produktausbeute. Durch die Zufuhr von Salzlösungen mehrwertiger Kationen während oder am Ende der Fermentation oder während oder am Ende einer Ultrafiltration oder Diafiltration oder während des Nanofiltrationsschritts und/oder ein Rückführen des Permeats in die Fermentationslösung werden die Ausbeuten an D-Pantothenat in Form mehrwertiger, bevorzugt zweiwertiger Ionen, wie Calcium oder Magnesium, weiter gesteigert.

Erfindungsgemäß vorteilhaft ist bei dem zuvor dargestellten Verfahren ferner die Reduzierung aufwendiger Aufarbeitungsschritte, insbesondere der Verzicht auf den Einsatz organischer Lösungsmittel, bei gleichzeitiger Bereitstellung eines gewünschten Produktes mit guter biologischer Wertigkeit. Ferner wird erfindungsgemäß die Menge an anfallendem Abwasser wesentlich reduziert. Dies resultiert somit in weiteren Einsparungen an aufwendigen Aufbereitungs- und Entsorgungsanlagen. Somit zeichnet sich das erfindungsgemäße Verfahren in vorteilhafter Weise dadurch aus, daß es einfacher, weniger störanfällig, weniger zeitaufwendig, deutlich kostengünstiger und damit wirtschaftlicher ist, als herkömmliche Verfahren.

Dies schließt jedoch nicht aus, daß das erfindungsgemäße Verfahren variiert werden kann. Das zuvor erläuterte erfindungsgemäße Verfahren kann durch einen oder mehrere der folgenden Verfahrensschritte ergänzt werden, die für sich genommen dem Fachmenschen vertraut sind. Hierbei sind alle denkbaren Kombinationen der nachfolgenden Verfahrensschritte mit den bislang genannten Verfahrensschritten erfindungsgemäß umfaßt.

So können die aus dem erfindungsgemäßen Verfahren resultierenden Lösungen z. B. durch Erhitzen (Sterilisation) oder andere Methoden, wie z.B. Pasteurisierung oder Sterilfiltration entkeimt werden.

In weiteren Varianten des erfindungsgemäßen Verfahrens kann vor der Trocknung und/oder Formulierung des Retentats wenigstens einer oder Kombinationen der nachfolgenden Schritte durchgeführt werden, umfassend Lyse und/oder Abtötung der Biomasse und/oder Abtrennung der Biomasse von der Fermentationslösung und/oder Zugabe weiterer Zuschlagstoffe und/oder Konzentrierung der Fermentationslösung, bevorzugt durch Wasserentzug.

Gegenstand der vorliegenden Erfindung ist somit auch ein Verfahren, wobei die Lyse und/oder Abtötung der Biomasse noch in der Fermentationslösung oder erst nach der Abtrennung der Biomasse von der Fermentationslösung durchgeführt wird. Dies kann beispielsweise durch eine Temperaturbehandlung, bevorzugt bei 80 - 200 °C und/oder eine Säurebehandlung, bevorzugt mit Schwefelsäure oder Salzsäure und/oder enzymatisch, bevorzugt mit Lysozym erfolgen.
Denkbar ist auch, daß die Abtrennung der enthaltenen Zellmasse direkt über die Nanofiltration, d.h. simultan mit dem Austausch von ein- gegen mehrwertige Kationen, erfolgt.

Die aus der Aufarbeitung über Nanofiltration resultierende Lösung kann vor der Trocknung und/oder Formulierung über einen geeigneten Verdampfer, z. B. Fallfilmverdampfer, Dünnschichtverdampfer oder Rotationsverdampfer aufkonzentriert werden. Solche Verdampfer werden z. B. von den Firmen GIG (4800 Attnang Puchheim, Österreich), GEA Canzler (52303 Düren, Deutschland), Diessel (31103 Hildesheim, Deutschland) und Pitton (35274 Kirchhain, Deutschland) hergestellt.

Um die farblichen Eigenschaften des Endproduktes zu verbessern, kann ein zusätzlicher Filtrationsschritt durchgeführt werden, bei dem den während des Verfahrens erhaltenen Lösungen etwas Aktivkohle zugesetzt wird und diese Suspension anschließend filtriert wird. Oder die während der Fermentation erhaltenen Lösungen können über ein kleines Aktivkohlebett geleitet werden. Die hierzu erforderlichen Mengen eingesetzter Aktivkohle liegen im Bereich weniger Gew.-% der Lösung und liegen im Wissen und Ermessen des Fachmanns.
Diese Filtrationen können erleichtert werden, indem der jeweiligen Lösung vor der Filtration ein handelsübliches Flockungshilfsmittel (z. B. Sedipur CF 902 oder Sedipur CL 930 der Firma BASF AG, Ludwigshafen) zusetzt wird.

In einer vorteilhaften Ausführungsform der vorliegenden Erfindung wird der Fermentationsaustrag (Fermentationsbrühe) durch Erhitzen sterilisiert und dann durch Zentrifugieren, Filtrieren, Ultrafiltrieren oder Dekantieren von der Zellmasse befreit. Nach Zusatz von 50 - 1000 mg/kg, bevorzugt 100- 200 mg/kg eines handelsüblichen Flockungshilfsmittels bezogen auf den Fermentationsaustrag wird über ein kurzes Bett von Aktivkohle und Sand filtriert, um eine Biomasse-freie Lösung mit hohem D-Pantothensäure-Gehalt zu erhalten. Anschließend wird diese aufbereitete Lösung durch Nanofiltration aufbereitet.

Die sich anschließende Trocknung dieser Lösung kann beispielsweise durch Sprühtrocknung erfolgen. Diese kann im Gleichstrom, Gegenstrom oder Mischstrom erfolgen. Zur Zerstäubung können alle bekannten Zerstäuber herangezogen werden, insbesondere Zentrifugalzerstäuber (Zerstäuberscheibe), Einstoffdüse oder Zweistoffdüse. Bevorzugte Trocknungstemperaturbedingungen sind 150 - 250 °C Turmeintrittstemperatur und 70 - 130 °C Turmaustrittstemperatur. Es kann aber auch bei höherem oder niedrigeren Temperatumiveau getrocknet werden. Um eine sehr niedrige Restfeuchte zu erzielen, kann noch ein weiterer Trocknungsschritt in einem Wirbelbett nachgeschaltet werden.

Die Sprühtrocknung läßt sich auch in einem FSD- oder SBD-Trockner (FSD: Fluidized Spray Dryer; SBD: Spray Bed Dryer), wie sie von den Firmen Niro (Kopenhagen, Dänemark) und APV-Anhydro (Kopenhagen, Dänemark) gebaut werden, durchführen, die eine Kombination von Sprühtrockner und Wirbelbett darstellen.
Bei der Sprühtrocknung kann ein Fließhilfsmittel zugesetzt werden. Dadurch können die Beläge an der Trocknerwandung reduziert und das Fließverhalten, gerade bei feinkörnigen Pulvern verbessert werden. Als Fließhilfsmittel kommen insbesondere Silikate, Stearate, Phosphate und Maisstärke in Betracht.
Prinzipiell kann die Trocknung auch in einer Sprühwirbelschicht erfolgen, wobei diese sowohl kontinuierlich als auch diskontinuierlich betrieben werden kann. Das Einsprühen der Lösung kann sowohl von oben (Topspray), von unten (Bottomspray) als auch von der Seite (Sidespray) erfolgen.

Gegenstand der vorliegenden Erfindung ist ferner eine Zusammensetzung für den Einsatz als Tierfutterzusatz und/oder Tierfutterergänzungsmittel, wobei sie herstellbar ist, indem
a) wenigstens ein D-Pantothensäure produzierender Mikroorganismus eingesetzt wird, dessen Pantothensäure-(pan)- und/oder Isoleucin/Valin-(ilv)-Biosynthese dereguliert ist und der wenigstens 2 g/l an Salzen der D-Pantothensäure durch Fermentation in einem Kulturmedium bildet, wobei dem Kulturmedium 0 - 20 g/l, bevorzugt 0 g/l freies β-Alanin und/oder β-Alanin-Salz zugeführt wird,
b) dem gebildeten D-Pantothenat Salze enthaltend mehrwertige Kationen zugeführt werden, wobei mehrwertige Salze der D-Pantothensäure ausgebildet werden,
c) die D-Pantothenat enthaltende Fermentationslösung durch Nanofiltration aufbereitet wird, wobei die mehrwertigen Salze der D-Pantothensäure angereichert werden,
d) das Retentat der Nanofiltration enthaltend mehrwertige Salze der D-Pantothensäure einer Trocknung und/oder Formulierung unterzogen wird.

In einer Variante der vorliegenden Erfindung ist eine Zusammensetzung umfaßt, die sich dadurch auszeichnet, daß sie herstellbar ist, indem vor der Nanofiltration in Schritt c) eine Abtrennung von Zellmasse oder von in der Lösung ausgefallenen Komponenten, bevorzugt durch Membranfiltration, besonders bevorzugt durch Ultrafiltration und ganz besonders bevorzugt durch Diafiltration erfolgt. Die vorliegende Erfindung betrifft ferner eine Zusammensetzung, die sich dadurch auszeichnet, daß sie herstellbar ist, indem während oder nach der Abtrennung von Zellmasse oder von in Lösung ausgefallenen Komponenten Salze (in fester Form oder als wässrige Lösung) enthaltend mehrwertige Kationen zugeführt werden. Erfindungsgemäß können diese Salze in einer weiteren Variante auch während der Nanofiltration zugeführt werden.

Erfindungsgemäß zeichnet sich die Zusammensetzung ferner dadurch aus, daß sie Salze der D-Pantothensäure in einer Konzentration von wenigstens 1-100 Gew.-%, bevorzugt 20-100 Gew.-% und besonders bevorzugt wenigstens 50 Gew.-% enthält. Gegenstand der vorliegenden Erfindung ist eine Zusammensetzung, die Salze der D-Pantothensäure in Form zweiwertiger Kationen, bevorzugt Calcium- und/oder Magnesium-D-Pantothenat enthält. Erfindungsgemäß bevorzugt ist eine Zusammensetzung, die sich dadurch auszeichnet, daß der Gehalt an Salzen der D-Pantothensäure in Form einwertiger Kationen ≤ 5 g/kg ist.

Erfindungsgemäß wird durch das zuvor beschriebene Verfahren ein Calcium- oder Magnesium-D-Pantothenat erhalten, das den Ansprüchen für einen Futtermittelzusatzstoff genügt. Diese Anforderungen sind beispielsweise ein relativ hoher Gehalt an D-Pantothenat und eine gute Verträglichkeit für den Zielorganismus sowie eine biologische Wertigkeit im Sinne der "Vitamin-Wirkung" des erfindungsgemäßen Produktes.

Die vorliegende Erfindung wird durch die nachfolgenden Beispiele näher erläutert, die jedoch nicht limitierend für die Erfindung sind:

### Beispiel 1:

In einem Laborfermenter mit Rührer und Begasungseinrichtung mit 14 I Inhalt wird wässriges Fermentationsmedium mit der folgenden Zusammensetzung vorgelegt:

| Einsatzstoff | Konzentration [g/l] |
|---|---|
| Hefeextrakt | 5 |
| Sojamehl | 40 |
| Natriumglutamat x H₂O | 5 |
| Ammoniumsulfat | 8 |
| KH₂PO₄ | 5 |
| K₂HPO₄ | 10 |
| NaH2PO4 x 2 H2O | 6,15 |
| Na2HPO4 x 2 H2O | 12 |

Nach der Sterilisation wurden folgende sterile Medienkomponenten zusätzlich zugegeben:

| Einsatzstoff | Konzentration [g/l] |
|---|---|
| Glucose x H2O | 20 |
| Calciumsulfat | 0,1 |
| Magnesiumsulfat | 1 |
| Natriumcitrat | 1 |
| FeSO₄ x 7 H₂O | 0,01 |
| Spurensalzlösung | 1 ml |

Die Spurensalzlösung setzte sich wie folgt zusammen :

0,15 g Na₂MoO₄ x 2 H₂O, 2,5 g H₃BO₃, 0,7 g CoCl₂ x 6 H₂O, 0,25 g CuSO₄ x 5 H₂O, 1,6 g MnCl₂ x 4 H₂O, 0,3 g ZnSO₄ x 7 H₂O wurden mit Wasser auf 1 l aufgefüllt. Die Zugabe der Spurensatztösung erfolgte über eine Sterilfiltration. Das Anfangsflüssigkeitsvolumen betrug 5 l. Die oben angeführten Gehalte sind auf diesen Wert bezogen.

Dieser Lösung wurden 100 ml Impfkultur (OD = 10) von Bacillus subtilis PA668 zugesetzt und bei 43 °C unter starkem Rühren bei einer Begasungsrate von 12 l/min fermentiert. Dieser Stamm ist gemäß der Anlage in der US 2004/0091979 beschrieben.

Innerhalb von 47 h wurden 2,1 l einer sterilen wässrigen Lösung zudosiert. Die Zusammensetzung war:

| Einsatzstoff | Konzentration [g/l] |
|---|---|
| Glucose | 800 |
| Calciumchlorid | 0,6 |
| Natriumglutamat x H₂O | 5 |
| Natriumcitrat | 2 |
| FeSO₄ x 7 H₂O | 0,2 |
| Spurensalzlösung | 6 ml |

Während der Fermentation wurde der pH Wert bei 7,2 durch die Zudosierung von 25 %iger Ammoniaklösung bzw. von 20 %iger Phosporsäure gehalten. Ammoniak dient gleichzeitig als Stickstoffquelle für die Fermentation. Die Drehzahl des Rührorgans wurde geregelt, indem der Gelöstsauerstoffgehalt auf 30% des Sättigungswertes gehalten wurde. Nach Abbruch der Zudosierung der Kohlenstoffquelle wurde die Fermentation so lange weitergeführt, bis der Gelöstsauerstoffgehalt (pO₂) einen Wert von 95% des Sättigungswerts erreicht hatte. Die Konzentration an D-Pantothenat bei Abbruch nach 48 h betrug 22,8 g/l.

In analoger Weise lassen sich auch Fermentationsbrühen erzeugen, die β-Alaninzufütterungsfrei Pantothensäure-Titer von über 20, 25, 30, 35, 40, 45, 50, 55, 60, 65, 70, 75, 80, 85, und >90 g/L aufweisen.

### Beispiel 2:

7000 ml des Fermentationsaustrages, hergestellt nach Beispiel 1, wurden einer Ultrazentrifugation unterzogen, wobei ein keramisches Monokanal-Rohrmodul (Fa. Atech, Gladbeck, Deutschland) eingesetzt wurde. Hierbei wurde zum einen eine Membran mit einer Porenweite von 20 kD (10nm) und mit einer Porenweite von 50nm eingesetzt.

Die Temperatur bei den Versuchen betrug 40 °C, die Überströmgeschwindigkeit 4 m/s und der Transmembrandruck (TMP= [p(Feed) + p(Retentat)]/2 - p(Permeat)), soweit nicht anders angegeben, 1 bar.

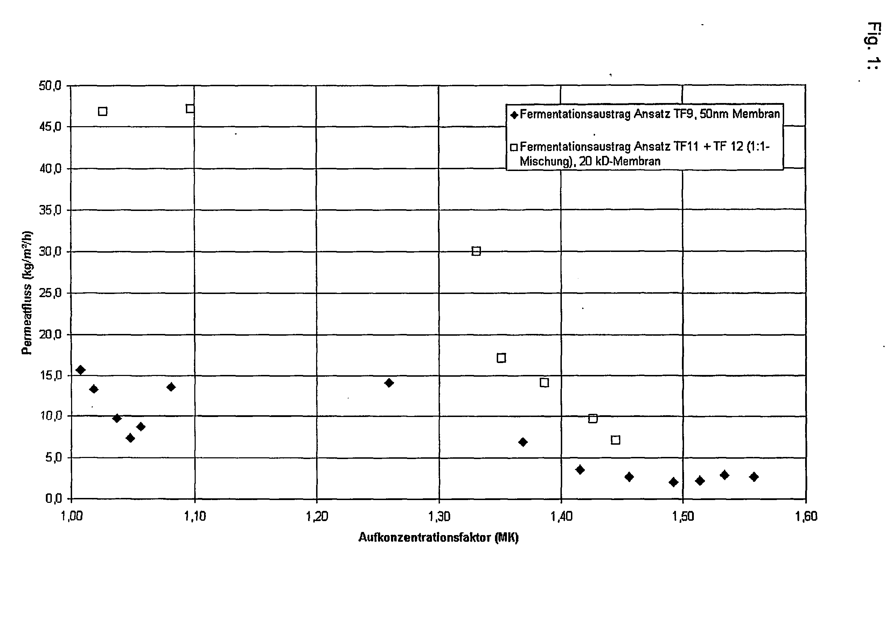

In Fig.1 sind die Transmembranflüsse (Permeatflüsse) in Abhängigkeit vom Aufkonzentrierungsfaktor MK (MK(t) = m_{Einsatz} / m_{Retentat} (t)) aufgetragen.

Dabei wird deutlich, daß die Membran mit der geringeren Porenweite (20 kD) deutlich höhere Flüsse als die Membran mit der größeren Porenweite (50 nm) zeigt.

### Beispiel 3:

7000 ml des Fermentationsaustrages, hergestellt nach Beispiel 1, wurden einer Ultrafiltration analog zu Beispiel 2 unterzogen, wobei die eingesetzte Membran eine Porenweite von 20 kD aufweist.

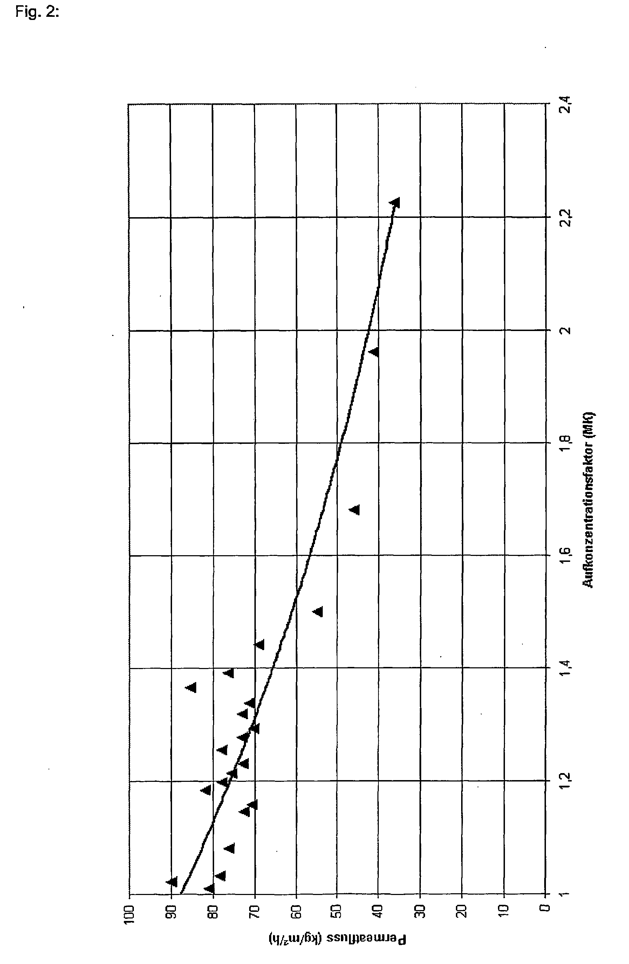

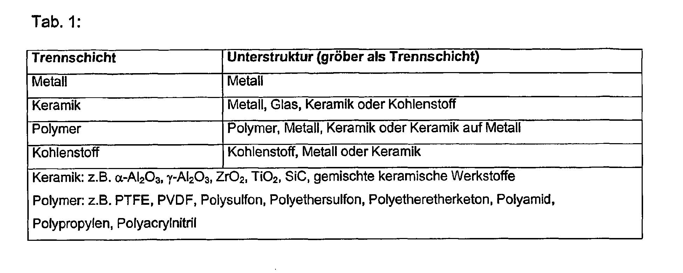

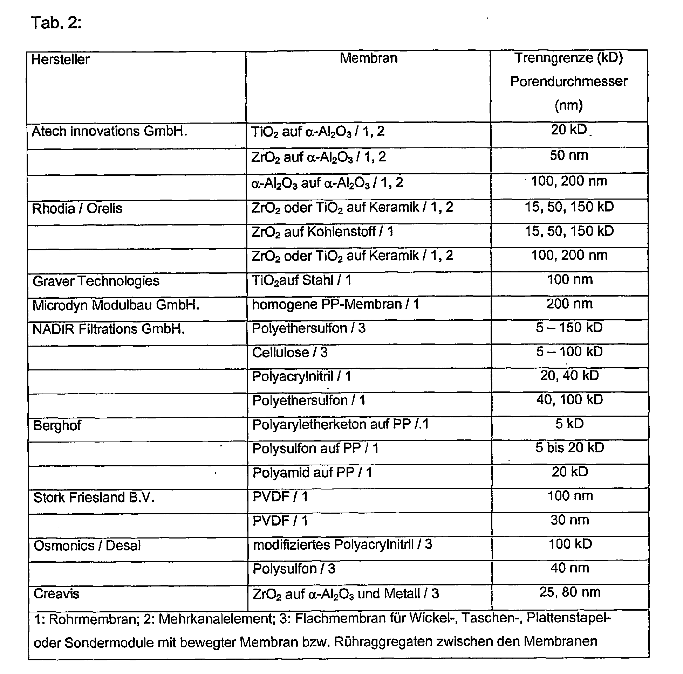

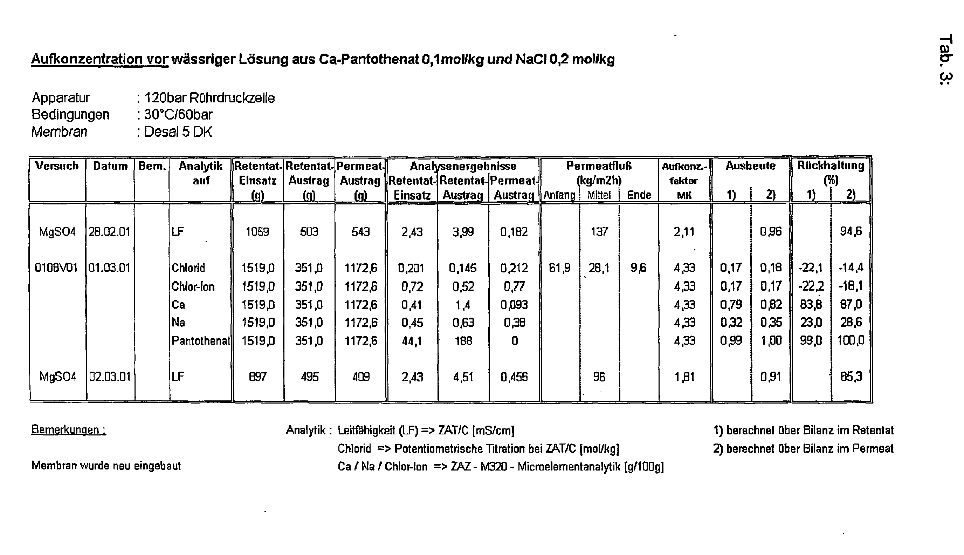

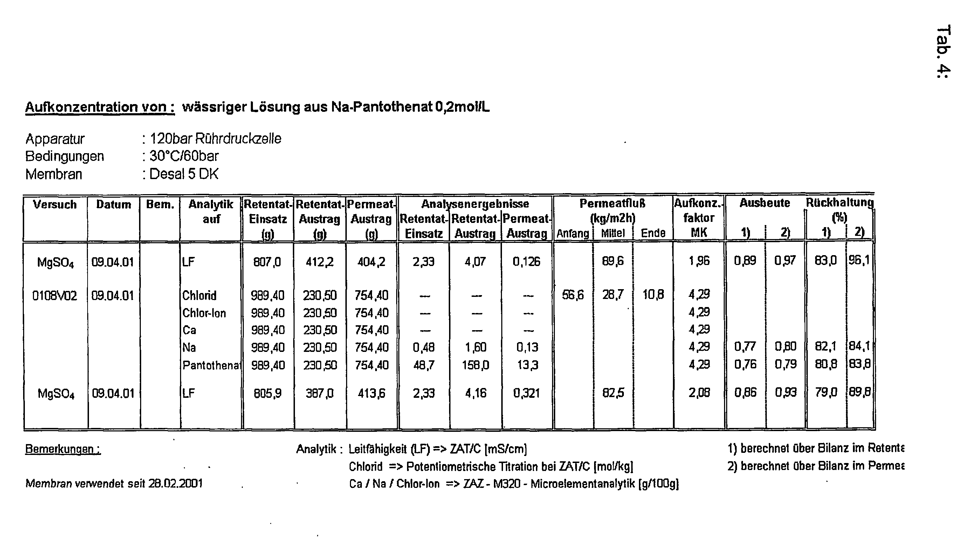

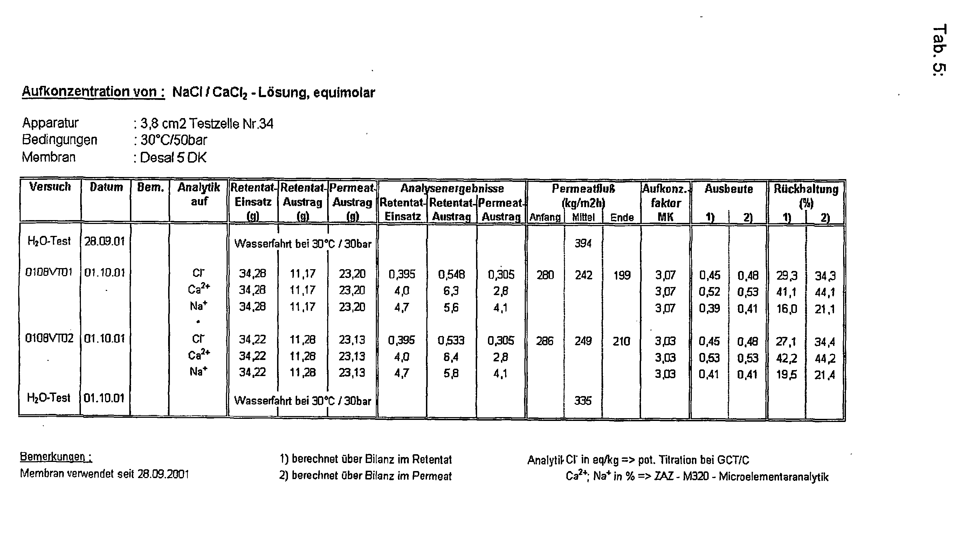

Fig. 2 zeigt, daß die Aufkonzentrierung mit bereits zentrifugiertem Fermentationsaustrag deutlich höher ist als in Beispiel 2.

### Beispiel 4:

1000 ml einer wässrigen Lösung enthaltend Calcium-Pantothenat (It. Tabelle 3, Spalte Retentat-Einsatz) wurden in eine Rührdruckzelle mit einem maximalen Betriebsinhalt von ca. 1,5 l eingesetzt. Der "Feed"-Druck wird bei dieser Zelle durch Aufpressen von Stickstoff erzeugt und die Überströmung der Membran durch Rühren mit einem über eine Magnetkupplung angetriebenen Ankerrührer sichergestellt.
Es wurde die Nanofiltrationsmembran DESAL 5 DK, bezogen von der Firma Osmonics Deutschland GmbH in Moers, verwendet.
Vor, zwischen und nach den Versuchen mit der o.g. Lösung wurden zur Überprüfung der Membranintegrität Rückhaltungstests mit MgSO₄-Lösung (2000 Gew.-ppm) durchgeführt.

Die Rückhaltung Rᵢ, ist in den beiden rechts stehenden Spalten der Tabelle 3 aufgeführt. Dabei ist die Rückhaltung wie folgt definiert: Rᵢ = 1 - c_{i,Permeat}/c_{i,Retentat}; mit Rᵢ = Rückhaltung für die Komponente i, c_{i,Permeat} = Konzentration der Komponente i im Permeat, c_{i,Retentat} = Konzentration der Komponente i im Retentat.
Mit den Konzentrationen sind die sich im instationären Versuch bei einem bestimmten Zeitpunkt einstellenden momentanen Konzentrationen gemeint, nicht aber die sich nach Versuchsende ergebenden Konzentrationen in den Fraktionen. Die Rückhaltung Rᵢ ist im Idealfall konzentrations-unabhängig, was bei der Berechnung der angegebenen Werte aus den Konzentrationen in den Fraktionen auch zugrunde gelegt wurde.
Aus Tabelle 3 geht hervor, daß Ca²⁺ und Pantothenat zu 84% bzw. 99% zurückgehalten werden, d.h. im Retentat vorliegen.

### Beispiel 5:

Bei der Aufarbeitung einer wässrigen Lösung aus Na-Pantothenat (0,2 mol/l) wurde unter analogen Bedingungen wie in Beispiel 4 eine Aufkonzentrierung durch Nanofiltration durchgeführt. Tabelle 4 zeigt, daß die Rückhaltung von Pantothenat bei 80% liegt.

### Beispiel 6:

Die Aufkonzentrierung einer äquimolaren Lösung von NaCl/CaCl₂ unter analogen Bedingungen wie in Beispiel 4 ist in Tabelle 5 zusammengefaßt. Hier ist zu erkennen, daß die Rückhaltung der Membran für Ca²⁺ mit 41% bzw. 42% relativ gering ist gegenüber der hohen Rückhaltung von Calcium in Verbindung mit Pantothenat (Beispiel 5).

Legende zu den Figuren und Tabellen
- Fig.: 1: Graphische Darstellung der Transmembranflüsse (Permeatflüsse) von Fermentationsaustrag bei einer Ultrafiltration in Abhängigkeit vom Aufkonzentrierungsfaktor MK unter Verwendung von Membranen mit einer Porenweite von 50 nm und 20 kD.
- Fig. 2:: Graphische Darstellung der Transmembranflüsse (Permeatflüsse) von zentrifugiertem Fermentationsaustrag bei einer Ultrafiltration in Abhängigkeit vom Aufkonzentrierungsfaktor MK unter Verwendung eine Membran mit einer Porenweite von 20 kD.
- Tab. 1:: Übersicht über asymmetrisch strukturierte Membranen zur Abtrennung von Zellmasse oder in Lösung ausgefallenen Komponenten.
- Tab.2:: Übersicht über Membranen und deren Eigenschaften zur Abtrennung von Zellmasse oder in Lösung ausgefallenen Komponenten.
- Tab. 3:: Übersicht über die Analysewerte einer Nanofiltration, insbesondere hinsichtlich der Rückhaltung von Calcium-lonen und Pantothenat, in einer wässrigen Lösung enthaltend 0,1 mol/kg Ca-Pantothenat und 0,2 mol/kg NaCl.
- Tab. 4:: Übersicht über die Analysewerte einer Nanofiltration, insbesondere hinsichtlich der Rückhaltung von Calcium-lonen und Pantothenat, in einer wässrigen Lösung enthaltend 0,2 mol/l Na-Pantothenat und 0,1 mol/l CaCl₂.
- Tab. 5:: Übersicht über die Analysewerte einer Nanofiltration, insbesondere hinsichtlich der Rückhaltung von Calcium-lonen, in einer wässrigen Lösung enthaltend äquimolare Mengen an NaCl und CaCl₂.

## Patentansprüche

1. Verfahren zur Herstellung von D-Pantothensäure und/oder deren Salze, **dadurch gekennzeichnet, daß**
a) wenigstens ein D-Pantothensäure produzierender Mikroorganismus eingesetzt wird, dessen Pantothensäure-(pan)- und/oder Isoleucin/Valin-(ilv)-Biosynthese dereguliert ist und der wenigstens 2 g/l an Salzen der D-Pantothensäure durch Fermentation in einem Kulturmedium bildet, wobei dem Kulturmedium 0 - 20 g/l freies β-Alanin und/oder β-Alanin-Salz zugeführt wird,
**b) zur Neutralisation der entstehenden Pantothensäure übliche Puffersysteme benutzt werden und** dem gebildeten D-Pantothenat Salze enthaltend mehrwertige Kationen zugeführt werden, wobei mehrwertige Salze der D-Pantothensäure ausgebildet werden,
c) die Lösung enthaltend mehrwertige Salze der D-Pantothensäure durch Nanofiltration aufbereitet wird, wobei die mehrwertigen Salze der D-Pantothensäure angereichert werden und
d) das Retentat der Nanofiltration enthaltend mehrwertige Salze der D-Pantothensäure einer Trocknung und/oder Formulierung unterzogen wird.

2. Verfahren gemäß Anspruch 1, **dadurch gekennzeichnet, daß** dem Kulturmedium kein freies β-Alanin und/oder β-Alanin-Salz zugeführt wird.

3. Verfahren gemäß einem der Ansprüche 1 oder 2, **dadurch gekennzeichnet, daß** als D-Pantothensäure produzierender Organismus ein Bakterium, eine Hefe oder ein Pilz eingesetzt wird.

4. Verfahren gemäß einem der Ansprüche 1-3, **dadurch gekennzeichnet, daß** als Mikroorganismus ein Bakterium aus der Familie der Bacillaceae eingesetzt wird.

5. Verfahren gemäß einem der Ansprüche 1-4, **dadurch gekennzeichnet, daß** ein Bakterium der Gattung Bacillus und bevorzugt der Art B. subtils, B. licheniformis oder B. amyloliquefaciens eingesetzt wird.

6. Verfahren gemäß einem der Ansprüche 1-5, **dadurch gekennzeichnet, daß** in Schritt a) ein Gehalt an D-Pantothensäure und/oder deren Salzen von wenigstens 10 g/l Kulturmedium, bevorzugt wenigstens 20 g/l, besonders bevorzugt wenigstens 40 g/l und höchst bevorzugt von wenigstens 60 g/l Kulturmedium gebildet wird.

7. Verfahren gemäß einem der Ansprüche 1-6, **dadurch gekennzeichnet, daß** vor der Nanofiltration in Schritt c) eine Abtrennung von Zellmasse oder von in der Lösung ausgefallenen Komponenten erfolgt.

8. Verfahren gemäß Anspruch 7, **dadurch gekennzeichnet, daß** die Abtrennung durch Dekantieren oder Membranfiltration, bevorzugt Ultrafiltration, erfolgt.

9. Verfahren gemäß einem der Ansprüche 7 oder 8, **dadurch gekennzeichnet, daß** die Membranfiltration als Diafiltration durchgeführt wird.

10. Verfahren gemäß einem der Ansprüche 1-9, **dadurch gekennzeichnet, daß** die Zugabe von Salzen enthaltend mehrwertige Kationen in fester Form oder wässriger Lösung während, bevorzugt am Ende, oder nach der Fermentation in Schritt a) oder während oder nach einer Membranfiltration einer D-Pantothenat-haltigen Lösung erfolgt.

11. Verfahren gemäß einem der Ansprüche 1-10, **dadurch gekennzeichnet, daß** die Zugabe von Salzen enthaltend mehrwertige Kationen in fester Form oder wässriger Lösung während einer Nanofiltration erfolgt.

12. Verfahren gemäß einem der Ansprüche 1-11, **dadurch gekennzeichnet, daß** die Zuführung einer wässrigen Lösung enthaltend mehrwertige Kationen kontinuierlich erfolgt.

13. Verfahren gemäß einem der Ansprüche 1-12, **dadurch gekennzeichnet, daß** die mehrwertigen Kationen als Calcium- und/oder Magnesium-Chlorid, -Nitrat, - Hydroxid, -Formiat, -Acetat, -Propionat, -Glycinat und/oder -Lactat zugesetzt werden.

14. Verfahren gemäß einem der Ansprüche 1-13, **dadurch gekennzeichnet, daß** als mehrwertiges Kation Ca²⁺ in einer Konzentration von 0,05 - 50 mol Ca²⁺/mol D-Pantothenat bevorzugt 0,2 - 2 mol Ca²⁺/mol D-Pantothenat zugeführt wird.

15. Verfahren gemäß einem der Ansprüche 1-14, **dadurch gekennzeichnet, daß** bei die Nanofiltration in Schritt c) eine Druckdifferenz über der Membran im Bereich von 5-100 bar, bevorzugt 20-80 bar und besonders bevorzugt bei 40-70 bar aufgebaut wird.

16. Verfahren gemäß einem der Ansprüche 1-15, **dadurch gekennzeichnet, daß** durch die Nanofiltration in Schritt c) der Gehalt an einwertigen Kationen, bevorzugt Ammonium-, Kalium- und/oder Natrium-Ionen, auf eine Konzentration von ≤ 5 g/kg Lösung reduziert wird.

17. Verfahren gemäß einem der Ansprüche 1-16, **dadurch gekennzeichnet, daß** das Permeat aus Schritt c) oder ein Teil davon in die Fermentation in Schritt a) zurückgeführt wird.

18. Verfahren gemäß einem der Ansprüche 1-17, **dadurch gekennzeichnet, daß** die Rückführung des Permeats oder von Teilen davon kontinuierlich erfolgt.

19. Verfahren gemäß einem der Ansprüche 1-18, **dadurch gekennzeichnet, daß** das Retentat aus Schritt c) eine Suspension enthaltend mehrwertige Salze der D-Pantothensäure ist.

## Claims

1. A process for preparing D-pantothenic acid and/or salts thereof, which comprises
a) using at least one D-pantothenic-acid-producing microorganism, the pantothenic acid (pan) and/or isoleucine/valine (ilv) biosynthesis of which is deregulated and which forms at least 2 g/l of salts of D-pantothenic acid by fermentation in a culture medium, with 0 - 20 g/l of free (β-alanine and/or β-alanine salt being fed to the culture medium,
b) customary buffer systems being used to neutralize the resulting pantothenic acid and salts containing polyvalent cations being fed to the D-pantothenate formed, polyvalent salts of D-pantothenic acid being formed,
c) the solution containing polyvalent salts of D-pantothenic acid being worked up by nanofiltration, the polyvalent salts of D-pantothenic acid being enriched and
d) the nanofiltration retentate containing polyvalent salts of D-pantothenic acid being subjected to drying and/or formulation.

2. The process according to claim 1, wherein free β-alanine and/or β-alanine salt is not fed to the culture medium.

3. The process according to either of claims 1 or 2, wherein the D-pantothenic-acid-producing organism used is a bacterium, a yeast or a fungus.

4. The process according to any one of claims 1-3, wherein the microorganism used is a bacterium from the Bacillaceae family.

5. The process according to any one of claims 1-4, wherein a bacterium of the genus Bacillus is used, and preferably of the species B. subtils [sic], B. licheniformis or B. amyloliquefaciens.

6. The process according to any one of claims 1-5, wherein, in step a), a content of D-pantothenic acid and/or salts thereof of at least 10 g/l of culture medium, preferably at least 20 g/l, particularly preferably at least 40 g/l, and very preferably at least 60 g/l, of culture medium is formed.

7. The process according to any one of claims 1-6, wherein cell mass or components precipitated out in the solution is/are separated off before the nanofiltration in step c).

8. The process according to claim 7, wherein the separation is carried out by decanting or membrane filtration, preferably ultrafiltration.

9. The process according to either of claims 7 or 8, wherein the membrane filtration is carried out as diafiltration.

10. The process according to any one of claims 1-9, wherein salts containing polyvalent cations are added in solid form or aqueous solution during, preferably at the end of, or after, the fermentation in step a) or during, or after, a membrane filtration of a D-pantothenate-containing solution.

11. The process according to any one of claims 1-10, wherein salts containing polyvalent cations are added in solid form or aqueous solution during a nanofiltration.

12. The process according to any one of claims 1-11, wherein an aqueous solution containing polyvalent cations is fed continuously.

13. The process according to any one of claims 1-12, wherein the polyvalent cations are added as calcium and/or magnesium chloride, nitrate, hydroxide, formate, acetate, propionate, glycinate and/or lactate.

14. The process according to any one of claims 1-13, wherein the polyvalent cation fed is Ca²⁺ at a concentration of 0.05-50 mol of Ca²⁺/mol of D-pantothenate, preferably 0.2-2 mol of Ca²⁺/mol of D-pantothenate.

15. The process according to any of claims 1-14, wherein, in the nanofiltration in step c), a pressure difference across the membrane in the range of 5-100 bar, preferably 20-80 bar, and particularly preferably 40-70 bar, is built up.

16. The process according to any one of claims 1-15, wherein the nanofiltration in step c) reduces the content of monovalent cations, preferably ammonium, potassium and/or sodium ions, to a concentration of ≤ 5 g/kg of solution.

17. The process according to any one of claims 1-16, wherein the permeate from step c) or a part thereof is recirculated to the fermentation in step a).

18. The process according to any one of claims 1-17, wherein the permeate or parts thereof are recirculated continuously.

19. The process according to any one of claims 1-18, wherein the retentate from c) is a suspension containing polyvalent salts of D-pantothenic acid.

## Revendications

1. Procédé de préparation d'acide D-pantothénique et/ou de ses sels, **caractérisé en ce que**
a) on met en oeuvre au moins un microorganisme produisant de l'acide D-pantothénique, dont la biosynthèse d'acide pantothénique (pan) et/ou d'isoleucine/valine (ilv) est dérégulée, et formant au moins 2 g/l de sels de l'acide D-pantothénique par fermentation dans un milieu de culture, et on ajoute au milieu de culture de 0 à 20 g/l de β-alanine libre et/ou d'un sel de β-alanine,
b) on utilise pour la neutralisation de l'acide pantothénique formé des systèmes tampons usuels et on ajoute au D-pantothénate formé des sels contenant des cations multivalents, avec formation de sels multivalents de l'acide D-pantothénique,
c) on traite la solution contenant des sels multivalents de l'acide D-pantothénique par nanofiltration, ce qui concentre les sels multivalents de l'acide D-pantothénique, et
d) on soumet le rétentat de la nanofiltration, contenant des sels multivalents de l'acide D-pantothénique, à un séchage et/ou une formulation.

2. Procédé suivant 1a revendication 1, **caractérisé en ce que** 1e milieu de culture n'est pas additionné de **β-**alanine libre et/ou de sel de β-alanine.

3. Procédé suivant l'une quelconque des revendications 1 ou 2, **caractérisé en ce que** l'on met en oeuvre en tant qu'organisme produisant de l'acide D-pantothénique une bactérie, une levure ou un champignon.

4. Procédé suivant l'une quelconque des revendications 1 à 3, **caractérisé en ce que** l'on met en oeuvre en tant que microorganisme une bactérie de la famille des bacillacées.

5. Procédé suivant l'une quelconque des revendications 1 à 4, **caractérisé en ce que** l'on met en oeuvre une bactérie du genre Bacillus et de préférence du type B. subtilis, B. licheniformis ou B. amyloliquefaciens.

6. Procédé suivant l'une quelconque des revendications 1 à 5, **caractérisé en ce que**, dans l'étape a), est formée une teneur en acide D-pantothénique et/ou en ses sels d'au moins 10 g/l de milieu de culture, de préférence d'au moins 20 g/l, plus préférablement d'au moins 40 g/l et le plus préférablement d'au moins 60 g/l de milieu de culture.

7. Procédé suivant l'une quelconque des revendications 1 à 6, **caractérisé en ce qu'**avant la nanofiltration dans l'étape c) a lieu une séparation de masse cellulaire ou de composants précipités dans la solution.

8. Procédé suivant la revendication 7, **caractérisé en ce que** la séparation est effectuée par décantation ou par filtration sur membrane, de préférence par ultrafiltration.

9. Procédé suivant l'une quelconque des revendications 7 ou 8, **caractérisé en ce que** la filtration sur membrane est entreprise en tant que diafiltration.

10. Procédé suivant l'une quelconque des revendications 1 à 9, **caractérisé en ce que** l'addition de sels contenant des cations multivalents se fait sous forme de solide ou de solution aqueuse, pendant, de préférence à la fin, ou après 1a fermentation de l'étape a), ou pendant ou après une filtration sur membrane d'une solution contenant du D-pantothénate.

11. Procédé suivant l'une quelconque des revendications 1 à 10, **caractérisé en ce que** l'addition de sels contenant des cations multivalents se fait sous forme de solide ou de solution aqueuse pendant une nanofiltration.

12. Procédé suivant l'une quelconque des revendications 1 à 11, **caractérisé en ce que** l'amenée d'une solution aqueuse contenant des cations multivalents se fait en continu.

13. Procédé suivant l'une quelconque des revendications 1 à 12, **caractérisé en ce que** les cations multivalents sont ajoutés en tant que chlorure, nitrate, hydroxyde, formiate, acétate, propionate, glycinate et/ou lactate de calcium et/ou de magnésium.

14. Procédé suivant l'une quelconque des revendications 1 à 13, **caractérisé en ce que** l'on ajoute en tant que cation multivalent du Ca²⁺ en une concentration de 0,05 à 50 moles de Ca²⁺/mole de D-pantothénate, de préférence de 0,2 à 2 moles de Ca²⁺/mole de D-pantothénate.

15. Procédé suivant l'une quelconque des revendications 1 à 14, **caractérisé en ce que**, lors de la nanofiltration dans l'étape c), il se crée une différence de pression sur la membrane de l'ordre de 5 à 100 bar, de préférence de 20 à 80 bar et plus préférablement de 40 à 70 bar.

16. Procédé suivant l'une quelconque des revendications 1 à 15, **caractérisé en ce que**, par la nanofiltration dans l'étape c), la teneur en cations monovalents, de préférence des ions ammonium, potassium et/ou sodium, est réduite à une concentration ≤ 5 g/kg de solution.

17. Procédé suivant l'une quelconque des revendications 1 à 16, **caractérisé en ce que** le perméat provenant de l'étape c) ou une partie de celui-ci est recyclé(e) dans la fermentation de l'étape a).

18. Procédé suivant l'une quelconque des revendications 1 à 17, **caractérisé en ce que** le recyclage du perméat ou de parties de celui-ci se fait en continu.

19. Procédé suivant l'une quelconque des revendications 1 à 18, **caractérisé en ce que** le rétentat de l'étape c) est une suspension contenant des sels multivalents de l'acide D-pantothénique.
